# FASCICULE DE BREVET EUROPEEN

(11) **EP 3 111 225 B1**
(45) Date de publication et mention de la délivrance du brevet: **28.07.2021**
(21) Numéro de dépôt: 15707611.8
(22) Date de dépôt: 27.02.2015
(51) Int. Cl.: G01N 33/68

(54) **BIOMARQUEURS POUR LE DIAGNOSTIC DE LA CARDIOMYOPATHIE DU PERIPARTUM**
DIAGNOSTISCHE BIOMARKER FÜR KARDIOMYOPATHIE WÄHREND DER GEBURT
DIAGNOSTIC BIOMARKERS FOR PERIPARTUM CARDIOMYOPATHY

(30) Priorité: 27.02.2014 FR 1451604
(43) Date de publication de la demande: 04.01.2017
(73) Titulaire: Assistance Publique - Hôpitaux de Paris, 75004 Paris (FR); University of Cape Town, 7701 Rondebosch (ZA); Université de Paris, 75006 Paris (FR)
(72) Inventeur: MEBAZAA, Alexandre, 92120 Montrouge (FR); SLIWA-HAHNLE, Karen, Cape Town (ZA); LAUNAY, Jean-Marie, 95100 Argenteuil (FR)
(74) Mandataire: Regimbeau
(86) Numéro de dépôt international: PCT/EP2015/054205
(87) Numéro de publication internationale: WO 2015/128494

(56) Documents cités:
- WO-A1-2009/079773
- WO-A1-2009/138451
- WO-A1-2012/106152
- WO-A2-2006/069373
- WO-A2-2010/060102
- US-A1- 2005 208 496
- US-A1- 2009 233 279
- IAN S. PATTEN ET AL: "Cardiac angiogenic imbalance leads to peripartum cardiomyopathy", NATURE, vol. 485, no. 7398, 9 mai 2012 (2012-05-09), pages 333-338, XP055153973, ISSN: 0028-0836, DOI: 10.1038/nature11040 cité dans la demande
- AFTAB A ANSARI ET AL: "Autoimmune Mechanisms as the Basis for Human Peripartum Cardiomyopathy", CLINICAL REVIEWS IN ALLERGY & IMMUNOLOGY, vol. 23, no. 3, 1 décembre 2002 (2002-12-01), pages 301-324, XP055154074, ISSN: 1080-0549, DOI: 10.1385/CRIAI:23:3:301
- Karen Sliwa-Hahnle: "ESC | Congresses | Heart Failure 2014 | Congress to You | Biomarker predicts Peripartum Cardiomyopathy", , 17 mai 2014 (2014-05-17), XP055155225, Extrait de l'Internet: URL:http://www.escardio.org/congresses/hea rt-failure-2014/congress-to-you/congress-n ews/Pages/biomarker-predicts-peripartum-ca rdiomyopathy.aspx [extrait le 2014-11-26]
- Anonymous: "ESC | About the ESC | ESC Press Office | ESC Press Releases | Biomarker test for Peripartum Cardiomyopathy could help reduce death after giving birth", , 17 mai 2014 (2014-05-17), XP055155224, Extrait de l'Internet: URL:http://www.escardio.org/about/press/pr ess-releases/pr-14/Pages/biomarker-test-pe ripartum-cardiomyopathy-could-help-reduce- death-after-giving-birth.aspx [extrait le 2014-11-26]
- VEGF: "GeneAnnot Search", , 1 January 2005 (2005-01-01), XP055682741, Retrieved from the Internet: URL:https://genecards.weizmann.ac.il/cgi-b in/geneannot/GA_search.pl?array=HG-U95&arr ay=HG-U133&array=HG-U133_Plus_2&keyword_ty pe=gene_symbol&keyword=vegfa&target=integr ated&.submit=Submit+Query [retrieved on 2020-04-03]
- Relaxin: "GeneAnnot Search", , 1 January 2005 (2005-01-01), XP055682753, Retrieved from the Internet: URL:https://genecards.weizmann.ac.il/cgi-b in/geneannot/GA_search.pl?array=HG-U95&arr ay=HG-U133&array=HG-U133_Plus_2&keyword_ty pe=gene_symbol&keyword=RLN1&target=integra ted&.submit=Submit+Query [retrieved on 2020-04-03]
- PIGF: "GeneAnnot Search", , 1 January 2005 (2005-01-01), XP055682933, Retrieved from the Internet: URL:https://genecards.weizmann.ac.il/cgi-b in/geneannot/GA_search.pl?array=HG-U95&arr ay=HG-U133&array=HG-U133_Plus_2&keyword_ty pe=gene_symbol&keyword=PIGF&target=integra ted&.submit=Submit+Query [retrieved on 2020-04-03]
- HAUSMAN G J ET AL: "Patterns of gene expression in pig adipose tissue: Insulin-like growth factor system proteins, neuropeptide Y (NPY), NPY receptors, neurotrophic factors and other secreted factors", DOMESTIC ANIMAL ENDOCRINOLOGY, ELSEVIER, AMSTERDAM, NL, vol. 35, no. 1 1 July 2008 (2008-07-01), pages 24-34, XP022707597, ISSN: 0739-7240, DOI: 10.1016/J.DOMANIEND.2008.01.004 [retrieved on 2008-02-12]

### SEQUENCE LISTING

<110> ASSISTANCE PUBLIQUE - HOPITAUX DE PARIS UNIVERSITY OF CAPE TOWN
<120> BIOMARQUEURS DIAGNOSTICS DE LA CARDIOMYOPATHIE DU PERIPARTUM
<130> B367266 D32917
<150> FR1451604
   <151> 2014-02-27
<160> 11
<170> Patent In version 3.5
<210> 1
   <211> 149
   <212> PRT
   <213> homo sapiens
<220>
   <221> MISC_FEATURE
   <223> PLGF
<400> 1
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg Pro Leu Arg Glu Lys Met Lys Pro Glu Arg Cys Gly Asp
       130                 135                 140
   Ala Val Pro Arg Arg
   145
<210> 2
   <211> 412
   <212> PRT
   <213> homo sapiens
<220>
   <221> MISC_FEATURE
   <223> VEGF isoforme A
<400> 2
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
                   85                  90                  95
   Glu Glu Glu Glu Lys Glu Glu Glu Arg Gly Pro Gln Trp Arg Leu Gly
               100                 105                 110
   Ala Arg Lys Pro Gly Ser Trp Thr Gly Glu Ala Ala Val Cys Ala Asp
           115                 120                 125
   Ser Ala Pro Ala Ala Arg Ala Pro Gln Ala Leu Ala Arg Ala Ser Gly
       130                 135                 140
   Arg Gly Gly Arg Val Ala Arg Arg Gly Ala Glu Glu Ser Gly Pro Pro
   145                 150                 155                 160
   His Ser Pro Ser Arg Arg Gly Ser Ala Ser Arg Ala Gly Pro Gly Arg
                   165                 170                 175
   Ala Ser Glu Thr Met Asn Phe Leu Leu Ser Trp Val His Trp Ser Leu
               180                 185                 190
   Ala Leu Leu Leu Tyr Leu His His Ala Lys Trp Ser Gln Ala Ala Pro
           195                 200                 205
   Met Ala Glu Gly Gly Gly Gln Asn His His Glu Val Val Lys Phe Met
       210                 215                 220
   Asp Val Tyr Gln Arg Ser Tyr Cys His Pro Ile Glu Thr Leu Val Asp
   225                 230                 235                 240
   Ile Phe Gln Glu Tyr Pro Asp Glu Ile Glu Tyr Ile Phe Lys Pro Ser
                   245                 250                 255
   Cys Val Pro Leu Met Arg Cys Gly Gly Cys Cys Asn Asp Glu Gly Leu
               260                 265                 270
   Glu Cys Val Pro Thr Glu Glu Ser Asn Ile Thr Met Gln Ile Met Arg
           275                 280                 285
   Ile Lys Pro His Gln Gly Gln His Ile Gly Glu Met Ser Phe Leu Gln
       290                 295                 300
   His Asn Lys Cys Glu Cys Arg Pro Lys Lys Asp Arg Ala Arg Gln Glu
   305                 310                 315                 320
   Lys Lys Ser Val Arg Gly Lys Gly Lys Gly Gln Lys Arg Lys Arg Lys
                   325                 330                 335
   Lys Ser Arg Tyr Lys Ser Trp Ser Val Tyr Val Gly Ala Arg Cys Cys
               340                 345                 350
   Leu Met Pro Trp Ser Leu Pro Gly Pro His Pro Cys Gly Pro Cys Ser
           355                 360                 365
   Glu Arg Arg Lys His Leu Phe Val Gln Asp Pro Gln Thr Cys Lys Cys
       370                 375                 380
   Ser Cys Lys Asn Thr Asp Ser Arg Cys Lys Ala Arg Gln Leu Glu Leu
   385                 390                 395                 400
   Asn Glu Arg Thr Cys Arg Cys Asp Lys Pro Arg Arg
                   405                 410
<210> 3
   <211> 395
   <212> PRT
   <213> homo sapiens
<220>
   <221> MISC_FEATURE
   <223> VEGF isoforme B
<400> 3
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
                   85                  90                  95
   Glu Glu Glu Glu Lys Glu Glu Glu Arg Gly Pro Gln Trp Arg Leu Gly
               100                 105                 110
   Ala Arg Lys Pro Gly Ser Trp Thr Gly Glu Ala Ala Val Cys Ala Asp
           115                 120                 125
   Ser Ala Pro Ala Ala Arg Ala Pro Gln Ala Leu Ala Arg Ala Ser Gly
       130                 135                 140
   Arg Gly Gly Arg Val Ala Arg Arg Gly Ala Glu Glu Ser Gly Pro Pro
   145                 150                 155                 160
   His Ser Pro Ser Arg Arg Gly Ser Ala Ser Arg Ala Gly Pro Gly Arg
                   165                 170                 175
   Ala Ser Glu Thr Met Asn Phe Leu Leu Ser Trp Val His Trp Ser Leu
               180                 185                 190
   Ala Leu Leu Leu Tyr Leu His His Ala Lys Trp Ser Gln Ala Ala Pro
           195                 200                 205
   Met Ala Glu Gly Gly Gly Gln Asn His His Glu Val Val Lys Phe Met
       210                 215                 220
   Asp Val Tyr Gln Arg Ser Tyr Cys His Pro Ile Glu Thr Leu Val Asp
   225 230 235 240
   Ile Phe Gln Glu Tyr Pro Asp Glu Ile Glu Tyr Ile Phe Lys Pro Ser
   245 250 255
   Cys Val Pro Leu Met Arg Cys Gly Gly Cys Cys Asn Asp Glu Gly Leu
   260 265 270
   Glu Cys Val Pro Thr Glu Glu Ser Asn Ile Thr Met Gln Ile Met Arg
   275 280 285
   Ile Lys Pro His Gln Gly Gln His Ile Gly Glu Met Ser Phe Leu Gln
   290 295 300
   His Asn Lys Cys Glu Cys Arg Pro Lys Lys Asp Arg Ala Arg Gln Glu
   305 310 315 320
   Lys Lys Ser Val Arg Gly Lys Gly Lys Gly Gln Lys Arg Lys Arg Lys
   325 330 335
   Lys Ser Arg Tyr Lys Ser Trp Ser Val Pro Cys Gly Pro Cys Ser Glu
   340 345 350
   Arg Arg Lys His Leu Phe Val Gln Asp Pro Gln Thr Cys Lys Cys Ser
   355 360 365
   Cys Lys Asn Thr Asp Ser Arg Cys Lys Ala Arg Gln Leu Glu Leu Asn
   370 375 380
   Glu Arg Thr Cys Arg Cys Asp Lys Pro Arg Arg
   385 390 395
<210> 4
   <211> 389
   <212> PRT
   <213> homo sapiens
<220>
   <221> MISC_FEATURE
   <223> VEGF isoforme C
<400> 4
   Met Thr Asp Arg Gln Thr Asp Thr Ala Pro Ser Pro Ser Tyr His Leu
   1 5 10 15
   Leu Pro Gly Arg Arg Arg Thr Val Asp Ala Ala Ala Ser Arg Gly Gln
   20 25 30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
                   85                  90                  95
   Glu Glu Glu Glu Lys Glu Glu Glu Arg Gly Pro Gln Trp Arg Leu Gly
               100                 105                 110
   Ala Arg Lys Pro Gly Ser Trp Thr Gly Glu Ala Ala Val Cys Ala Asp
           115                 120                 125
   Ser Ala Pro Ala Ala Arg Ala Pro Gln Ala Leu Ala Arg Ala Ser Gly
       130                 135                 140
   Arg Gly Gly Arg Val Ala Arg Arg Gly Ala Glu Glu Ser Gly Pro Pro
   145                 150                 155                 160
   His Ser Pro Ser Arg Arg Gly Ser Ala Ser Arg Ala Gly Pro Gly Arg
                   165                 170                 175
   Ala Ser Glu Thr Met Asn Phe Leu Leu Ser Trp Val His Trp Ser Leu
               180                 185                 190
   Ala Leu Leu Leu Tyr Leu His His Ala Lys Trp Ser Gln Ala Ala Pro
           195                 200                 205
   Met Ala Glu Gly Gly Gly Gln Asn His His Glu Val Val Lys Phe Met
       210                 215                 220
   Asp Val Tyr Gln Arg Ser Tyr Cys His Pro Ile Glu Thr Leu Val Asp
   225                 230                 235                 240
   Ile Phe Gln Glu Tyr Pro Asp Glu Ile Glu Tyr Ile Phe Lys Pro Ser
                   245                 250                 255
   Cys Val Pro Leu Met Arg Cys Gly Gly Cys Cys Asn Asp Glu Gly Leu
               260                 265                 270
   Glu Cys Val Pro Thr Glu Glu Ser Asn Ile Thr Met Gln Ile Met Arg
           275                 280                 285
   Ile Lys Pro His Gln Gly Gln His Ile Gly Glu Met Ser Phe Leu Gln
       290                 295                 300
   His Asn Lys Cys Glu Cys Arg Pro Lys Lys Asp Arg Ala Arg Gln Glu
   305                 310                 315                 320
   Lys Lys Ser Val Arg Gly Lys Gly Lys Gly Gln Lys Arg Lys Arg Lys
                   325                 330                 335
   Lys Ser Arg Pro Cys Gly Pro Cys Ser Glu Arg Arg Lys His Leu Phe
               340                 345                 350
   Val Gln Asp Pro Gln Thr Cys Lys Cys Ser Cys Lys Asn Thr Asp Ser
           355                 360                 365
   Arg Cys Lys Ala Arg Gln Leu Glu Leu Asn Glu Arg Thr Cys Arg Cys
       370                 375                 380
   Asp Lys Pro Arg Arg
   385
<210> 5
   <211> 371
   <212> PRT
   <213> homo sapiens
<220>
   <221> MISC_FEATURE
   <223> VEGF isoforme D
<400> 5
   Met Thr Asp Arg Gln Thr Asp Thr Ala Pro Ser Pro Ser Tyr His Leu
   1               5                   10                  15
   Leu Pro Gly Arg Arg Arg Thr Val Asp Ala Ala Ala Ser Arg Gly Gln
               20                  25                  30
   Gly Pro Glu Pro Ala Pro Gly Gly Gly Val Glu Gly Val Gly Ala Arg
           35                  40                  45
   Gly Val Ala Leu Lys Leu Phe Val Gln Leu Leu Gly Cys Ser Arg Phe
       50                  55                  60
   Gly Gly Ala Val Val Arg Ala Gly Glu Ala Glu Pro Ser Gly Ala Ala
   65                  70                  75                  80
   Arg Ser Ala Ser Ser Gly Arg Glu Glu Pro Gln Pro Glu Glu Gly Glu
   85 90 95
   Glu Glu Glu Glu Lys Glu Glu Glu Arg Gly Pro Gln Trp Arg Leu Gly
   100 105 110
   Ala Arg Lys Pro Gly Ser Trp Thr Gly Glu Ala Ala Val Cys Ala Asp
   115 120 125
   Ser Ala Pro Ala Ala Arg Ala Pro Gln Ala Leu Ala Arg Ala Ser Gly
   130 135 140
   Arg Gly Gly Arg Val Ala Arg Arg Gly Ala Glu Glu Ser Gly Pro Pro
   145 150 155 160
   His Ser Pro Ser Arg Arg Gly Ser Ala Ser Arg Ala Gly Pro Gly Arg
   165 170 175
   Ala Ser Glu Thr Met Asn Phe Leu Leu Ser Trp Val His Trp Ser Leu
   180 185 190
   Ala Leu Leu Leu Tyr Leu His His Ala Lys Trp Ser Gln Ala Ala Pro
   195 200 205
   Met Ala Glu Gly Gly Gly Gln Asn His His Glu Val Val Lys Phe Met
   210 215 220
   Asp Val Tyr Gln Arg Ser Tyr Cys His Pro Ile Glu Thr Leu Val Asp
   225 230 235 240
   Ile Phe Gln Glu Tyr Pro Asp Glu Ile Glu Tyr Ile Phe Lys Pro Ser
   245 250 255
   Cys Val Pro Leu Met Arg Cys Gly Gly Cys Cys Asn Asp Glu Gly Leu
   260 265 270
   Glu Cys Val Pro Thr Glu Glu Ser Asn Ile Thr Met Gln Ile Met Arg
   275 280 285
   Ile Lys Pro His Gln Gly Gln His Ile Gly Glu Met Ser Phe Leu Gln
   290 295 300
   His Asn Lys Cys Glu Cys Arg Pro Lys Lys Asp Arg Ala Arg Gln Glu
   305 310 315 320
   Asn Pro Cys Gly Pro Cys Ser Glu Arg Arg Lys His Leu Phe Val Gln
   325 330 335
   Asp Pro Gln Thr Cys Lys Cys Ser Cys Lys Asn Thr Asp Ser Arg Cys
   340 345 350
   Lys Ala Arg Gln Leu Glu Leu Asn Glu Arg Thr Cys Arg Cys Asp Lys
   355 360 365
   Pro Arg Arg
   370
<210> 6
   <211> 354
   <212> PRT
   <213> homo sapiens
<220>
   <221> MISC_FEATURE
   <223> VEGF isoforme E
<400> 6
   Met Thr Asp Arg Gln Thr Asp Thr Ala Pro Ser Pro Ser Tyr His Leu
   1 5 10 15
   Leu Pro Gly Arg Arg Arg Thr Val Asp Ala Ala Ala Ser Arg Gly Gln
   20 25 30
   Gly Pro Glu Pro Ala Pro Gly Gly Gly Val Glu Gly Val Gly Ala Arg
   35 40 45
   Gly Val Ala Leu Lys Leu Phe Val Gln Leu Leu Gly Cys Ser Arg Phe
   50 55 60
   Gly Gly Ala Val Val Arg Ala Gly Glu Ala Glu Pro Ser Gly Ala Ala
   65 70 75 80
   Arg Ser Ala Ser Ser Gly Arg Glu Glu Pro Gln Pro Glu Glu Gly Glu
   85 90 95
   Glu Glu Glu Glu Lys Glu Glu Glu Arg Gly Pro Gln Trp Arg Leu Gly
   100 105 110
   Ala Arg Lys Pro Gly Ser Trp Thr Gly Glu Ala Ala Val Cys Ala Asp
   115 120 125
   Ser Ala Pro Ala Ala Arg Ala Pro Gln Ala Leu Ala Arg Ala Ser Gly
   130 135 140
   Arg Gly Gly Arg Val Ala Arg Arg Gly Ala Glu Glu Ser Gly Pro Pro
   145 150 155 160
   His Ser Pro Ser Arg Arg Gly Ser Ala Ser Arg Ala Gly Pro Gly Arg
   165 170 175
   Ala Ser Glu Thr Met Asn Phe Leu Leu Ser Trp Val His Trp Ser Leu
   180 185 190
   Ala Leu Leu Leu Tyr Leu His His Ala Lys Trp Ser Gln Ala Ala Pro
   195 200 205
   Met Ala Glu Gly Gly Gly Gln Asn His His Glu Val Val Lys Phe Met
   210 215 220
   Asp Val Tyr Gln Arg Ser Tyr Cys His Pro Ile Glu Thr Leu Val Asp
   225 230 235 240
   Ile Phe Gln Glu Tyr Pro Asp Glu Ile Glu Tyr Ile Phe Lys Pro Ser
   245 250 255
   Cys Val Pro Leu Met Arg Cys Gly Gly Cys Cys Asn Asp Glu Gly Leu
   260 265 270
   Glu Cys Val Pro Thr Glu Glu Ser Asn Ile Thr Met Gln Ile Met Arg
   275 280 285
   Ile Lys Pro His Gln Gly Gln His Ile Gly Glu Met Ser Phe Leu Gln
   290 295 300
   His Asn Lys Cys Glu Cys Arg Pro Lys Lys Asp Arg Ala Arg Gln Glu
   305 310 315 320
   Asn Pro Cys Gly Pro Cys Ser Glu Arg Arg Lys His Leu Phe Val Gln
   325 330 335
   Asp Pro Gln Thr Cys Lys Cys Ser Cys Lys Asn Thr Asp Ser Arg Cys
   340 345 350
   Lys Met
<210> 7
   <211> 541
   <212> PRT
   <213> homo sapiens
<220>
   <221> MISC_FEATURE
   <223> sFlt-1 isoforme 4
<400> 7
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
   245 250 255
   Pro Leu Asn Thr Arg Val Gln Met Thr Trp Ser Tyr Pro Asp Glu Lys
   260 265 270
   Asn Lys Arg Ala Ser Val Arg Arg Arg Ile Asp Gln Ser Asn Ser His
   275 280 285
   Ala Asn Ile Phe Tyr Ser Val Leu Thr Ile Asp Lys Met Gln Asn Lys
   290 295 300
   Asp Lys Gly Leu Tyr Thr Cys Arg Val Arg Ser Gly Pro Ser Phe Lys
   305 310 315 320
   Ser Val Asn Thr Ser Val His Ile Tyr Asp Lys Ala Phe Ile Thr Val
   325 330 335
   Lys His Arg Lys Gln Gln Val Leu Glu Thr Val Ala Gly Lys Arg Ser
   340 345 350
   Tyr Arg Leu Ser Met Lys Val Lys Ala Phe Pro Ser Pro Glu Val Val
   355 360 365
   Trp Leu Lys Asp Gly Leu Pro Ala Thr Glu Lys Ser Ala Arg Tyr Leu
   370 375 380
   Thr Arg Gly Tyr Ser Leu Ile Ile Lys Asp Val Thr Glu Glu Asp Ala
   385 390 395 400
   Gly Asn Tyr Thr Ile Leu Leu Ser Ile Lys Gln Ser Asn Val Phe Lys
   405 410 415
   Asn Leu Thr Ala Thr Leu Ile Val Asn Val Lys Pro Gln Ile Tyr Glu
   420 425 430
   Lys Ala Val Ser Ser Phe Pro Asp Pro Ala Leu Tyr Pro Leu Gly Ser
   435 440 445
   Arg Gln Ile Leu Thr Cys Thr Ala Tyr Gly Ile Pro Gln Pro Thr Ile
   450 455 460
   Lys Trp Phe Trp His Pro Cys Asn His Asn His Ser Glu Ala Arg Cys
   465 470 475 480
   Asp Phe Cys Ser Asn Asn Glu Glu Ser Phe Ile Leu Asp Ala Asp Ser
   485 490 495
   Asn Met Gly Asn Arg Ile Glu Ser Ile Thr Gln Arg Met Ala Ile Ile
   500 505 510
   Glu Gly Lys Asn Lys Leu Pro Pro Ala Asn Ser Ser Phe Met Leu Pro
   515 520 525
   Pro Thr Ser Phe Ser Ser Asn Tyr Phe His Phe Leu Pro
   530 535 540
<210> 8
   <211> 733
   <212> PRT
   <213> homo sapiens
<220>
   <221> MISC_FEATURE
   <223> sFlt-1 isoforme 3
<400> 8
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Lys Leu Lys Asp Pro
   20 25 30
   Glu Leu Ser Leu Lys Gly Thr Gln His Ile Met Gln Ala Gly Gln Thr
   35 40 45
   Leu His Leu Gln Cys Arg Gly Glu Ala Ala His Lys Trp Ser Leu Pro
   50 55 60
   Glu Met Val Ser Lys Glu Ser Glu Arg Leu Ser Ile Thr Lys Ser Ala
   65 70 75 80
   Cys Gly Arg Asn Gly Lys Gln Phe Cys Ser Thr Leu Thr Leu Asn Thr
   85 90 95
   Ala Gln Ala Asn His Thr Gly Phe Tyr Ser Cys Lys Tyr Leu Ala Val
   100 105 110
   Pro Thr Ser Lys Lys Lys Glu Thr Glu Ser Ala Ile Tyr Ile Phe Ile
   115 120 125
   Ser Asp Thr Gly Arg Pro Phe Val Glu Met Tyr Ser Glu Ile Pro Glu
   130 135 140
   Ile Ile His Met Thr Glu Gly Arg Glu Leu Val Ile Pro Cys Arg Val
   145 150 155 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Arg Ala Ser Val Arg Arg Arg Ile Asp Gln Ser Asn Ser His
           275                 280                 285
   Ala Asn Ile Phe Tyr Ser Val Leu Thr Ile Asp Lys Met Gln Asn Lys
       290                 295                 300
   Asp Lys Gly Leu Tyr Thr Cys Arg Val Arg Ser Gly Pro Ser Phe Lys
   305                 310                 315                 320
   Ser Val Asn Thr Ser Val His Ile Tyr Asp Lys Ala Phe Ile Thr Val
                   325                 330                 335
   Lys His Arg Lys Gln Gln Val Leu Glu Thr Val Ala Gly Lys Arg Ser
               340                 345                 350
   Tyr Arg Leu Ser Met Lys Val Lys Ala Phe Pro Ser Pro Glu Val Val
           355                 360                 365
   Trp Leu Lys Asp Gly Leu Pro Ala Thr Glu Lys Ser Ala Arg Tyr Leu
       370                 375                 380
   Thr Arg Gly Tyr Ser Leu Ile Ile Lys Asp Val Thr Glu Glu Asp Ala
   385                 390                 395                 400
   Gly Asn Tyr Thr Ile Leu Leu Ser Ile Lys Gln Ser Asn Val Phe Lys
                   405                 410                 415
   Asn Leu Thr Ala Thr Leu Ile Val Asn Val Lys Pro Gln Ile Tyr Glu
               420                 425                 430
   Lys Ala Val Ser Ser Phe Pro Asp Pro Ala Leu Tyr Pro Leu Gly Ser
           435                 440                 445
   Arg Gln Ile Leu Thr Cys Thr Ala Tyr Gly Ile Pro Gln Pro Thr Ile
       450                 455                 460
   Lys Trp Phe Trp His Pro Cys Asn His Asn His Ser Glu Ala Arg Cys
   465                 470                 475                 480
   Asp Phe Cys Ser Asn Asn Glu Glu Ser Phe Ile Leu Asp Ala Asp Ser
                   485                 490                 495
   Asn Met Gly Asn Arg Ile Glu Ser Ile Thr Gln Arg Met Ala Ile Ile
               500                 505                 510
   Glu Gly Lys Asn Lys Met Ala Ser Thr Leu Val Val Ala Asp Ser Arg
           515                 520                 525
   Ile Ser Gly Ile Tyr Ile Cys Ile Ala Ser Asn Lys Val Gly Thr Val
       530                 535                 540
   Gly Arg Asn Ile Ser Phe Tyr Ile Thr Asp Val Pro Asn Gly Phe His
   545                 550                 555                 560
   Val Asn Leu Glu Lys Met Pro Thr Glu Gly Glu Asp Leu Lys Leu Ser
                   565                 570                 575
   Cys Thr Val Asn Lys Phe Leu Tyr Arg Asp Val Thr Trp Ile Leu Leu
               580                 585                 590
   Arg Thr Val Asn Asn Arg Thr Met His Tyr Ser Ile Ser Lys Gln Lys
           595                 600                 605
   Met Ala Ile Thr Lys Glu His Ser Ile Thr Leu Asn Leu Thr Ile Met
       610                 615                 620
   Asn Val Ser Leu Gln Asp Ser Gly Thr Tyr Ala Cys Arg Ala Arg Asn
   625                 630                 635                 640
   Val Tyr Thr Gly Glu Glu Ile Leu Gln Lys Lys Glu Ile Thr Ile Arg
                   645                 650                 655
   Asp Gln Glu Ala Pro Tyr Leu Leu Arg Asn Leu Ser Asp His Thr Val
                   660                 665                 670
   Ala Ile Ser Ser Ser Thr Thr Leu Asp Cys His Ala Asn Gly Val Pro
           675                 680                 685
   Glu Pro Gln Ile Thr Trp Phe Lys Asn Asn His Lys Ile Gln Gln Glu
       690                 695                 700
   Pro Glu Leu Tyr Thr Ser Thr Ser Pro Ser Ser Ser Ser Ser Ser Pro
   705                 710                 715                 720
   Leu Ser Ser Ser Ser Ser Ser Ser Ser Ser Ser Ser Ser
                   725                 730
<210> 9
   <211> 687
   <212> PRT
   <213> homo sapiens
<220>
   <221> MISC_FEATURE
   <223> sFlt-1 isoforme 2
<400> 9
   Met Val Ser Tyr Trp Asp Thr Gly Val Leu Leu Cys Ala Leu Leu Ser
   1               5                   10                  15
   Cys Leu Leu Leu Thr Gly Ser Ser Ser Gly Ser Lys Leu Lys Asp Pro
               20                  25                  30
   Glu Leu Ser Leu Lys Gly Thr Gln His Ile Met Gln Ala Gly Gln Thr
           35                  40                  45
   Leu His Leu Gln Cys Arg Gly Glu Ala Ala His Lys Trp Ser Leu Pro
       50                  55                  60
   Glu Met Val Ser Lys Glu Ser Glu Arg Leu Ser Ile Thr Lys Ser Ala
   65                  70                  75                  80
   Cys Gly Arg Asn Gly Lys Gln Phe Cys Ser Thr Leu Thr Leu Asn Thr
                   85                  90                  95
   Ala Gln Ala Asn His Thr Gly Phe Tyr Ser Cys Lys Tyr Leu Ala Val
               100                 105                 110
   Pro Thr Ser Lys Lys Lys Glu Thr Glu Ser Ala Ile Tyr Ile Phe Ile
           115                 120                 125
   Ser Asp Thr Gly Arg Pro Phe Val Glu Met Tyr Ser Glu Ile Pro Glu
       130                 135                 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Arg Ala Ser Val Arg Arg Arg Ile Asp Gln Ser Asn Ser His
           275                 280                 285
   Ala Asn Ile Phe Tyr Ser Val Leu Thr Ile Asp Lys Met Gln Asn Lys
       290                 295                 300
   Asp Lys Gly Leu Tyr Thr Cys Arg Val Arg Ser Gly Pro Ser Phe Lys
   305                 310                 315                 320
   Ser Val Asn Thr Ser Val His Ile Tyr Asp Lys Ala Phe Ile Thr Val
                   325                 330                 335
   Lys His Arg Lys Gln Gln Val Leu Glu Thr Val Ala Gly Lys Arg Ser
               340                 345                 350
   Tyr Arg Leu Ser Met Lys Val Lys Ala Phe Pro Ser Pro Glu Val Val
           355                 360                 365
   Trp Leu Lys Asp Gly Leu Pro Ala Thr Glu Lys Ser Ala Arg Tyr Leu
       370                 375                 380
   Thr Arg Gly Tyr Ser Leu Ile Ile Lys Asp Val Thr Glu Glu Asp Ala
   385                 390                 395                 400
   Gly Asn Tyr Thr Ile Leu Leu Ser Ile Lys Gln Ser Asn Val Phe Lys
                   405                 410                 415
   Asn Leu Thr Ala Thr Leu Ile Val Asn Val Lys Pro Gln Ile Tyr Glu
               420                 425                 430
   Lys Ala Val Ser Ser Phe Pro Asp Pro Ala Leu Tyr Pro Leu Gly Ser
           435                 440                 445
   Arg Gln Ile Leu Thr Cys Thr Ala Tyr Gly Ile Pro Gln Pro Thr Ile
       450                 455                 460
   Lys Trp Phe Trp His Pro Cys Asn His Asn His Ser Glu Ala Arg Cys
   465                 470                 475                 480
   Asp Phe Cys Ser Asn Asn Glu Glu Ser Phe Ile Leu Asp Ala Asp Ser
                   485                 490                 495
   Asn Met Gly Asn Arg Ile Glu Ser Ile Thr Gln Arg Met Ala Ile Ile
               500                 505                 510
   Glu Gly Lys Asn Lys Met Ala Ser Thr Leu Val Val Ala Asp Ser Arg
           515                 520                 525
   Ile Ser Gly Ile Tyr Ile Cys Ile Ala Ser Asn Lys Val Gly Thr Val
       530                 535                 540
   Gly Arg Asn Ile Ser Phe Tyr Ile Thr Asp Val Pro Asn Gly Phe His
   545                 550                 555                 560
   Val Asn Leu Glu Lys Met Pro Thr Glu Gly Glu Asp Leu Lys Leu Ser
                   565                 570                 575
   Cys Thr Val Asn Lys Phe Leu Tyr Arg Asp Val Thr Trp Ile Leu Leu
               580                 585                 590
   Arg Thr Val Asn Asn Arg Thr Met His Tyr Ser Ile Ser Lys Gln Lys
           595                 600                 605
   Met Ala Ile Thr Lys Glu His Ser Ile Thr Leu Asn Leu Thr Ile Met
       610                 615                 620
   Asn Val Ser Leu Gln Asp Ser Gly Thr Tyr Ala Cys Arg Ala Arg Asn
   625                 630                 635                 640
   Val Tyr Thr Gly Glu Glu Ile Leu Gln Lys Lys Glu Ile Thr Ile Arg
   645 650 655
   Gly Glu His Cys Asn Lys Lys Ala Val Phe Ser Arg Ile Ser Lys Phe
   660 665 670
   Lys Ser Thr Arg Asn Asp Cys Thr Thr Gln Ser Asn Val Lys His
   675 680 685
<210> 10
   <211> 1338
   <212> PRT
   <213> homo sapiens
<220>
   <221> MISC_FEATURE
   <223> sFlt-1 isoforme 1
<400> 10
   Met Val Ser Tyr Trp Asp Thr Gly Val Leu Leu Cys Ala Leu Leu Ser
   1 5 10 15
   Cys Leu Leu Leu Thr Gly Ser Ser Ser Gly Ser Lys Leu Lys Asp Pro
   20 25 30
   Glu Leu Ser Leu Lys Gly Thr Gln His Ile Met Gln Ala Gly Gln Thr
   35 40 45
   Leu His Leu Gln Cys Arg Gly Glu Ala Ala His Lys Trp Ser Leu Pro
   50 55 60
   Glu Met Val Ser Lys Glu Ser Glu Arg Leu Ser Ile Thr Lys Ser Ala
   65 70 75 80
   Cys Gly Arg Asn Gly Lys Gln Phe Cys Ser Thr Leu Thr Leu Asn Thr
   85 90 95
   Ala Gln Ala Asn His Thr Gly Phe Tyr Ser Cys Lys Tyr Leu Ala Val
   100 105 110
   Pro Thr Ser Lys Lys Lys Glu Thr Glu Ser Ala Ile Tyr Ile Phe Ile
   115 120 125
   Ser Asp Thr Gly Arg Pro Phe Val Glu Met Tyr Ser Glu Ile Pro Glu
   130 135 140
   Ile Ile His Met Thr Glu Gly Arg Glu Leu Val Ile Pro Cys Arg Val
   145                 150                 155                 160
   Thr Ser Pro Asn Ile Thr Val Thr Leu Lys Lys Phe Pro Leu Asp Thr
                   165                 170                 175
   Leu Ile Pro Asp Gly Lys Arg Ile Ile Trp Asp Ser Arg Lys Gly Phe
               180                 185                 190
   Ile Ile Ser Asn Ala Thr Tyr Lys Glu Ile Gly Leu Leu Thr Cys Glu
           195                 200                 205
   Ala Thr Val Asn Gly His Leu Tyr Lys Thr Asn Tyr Leu Thr His Arg
       210                 215                 220
   Gln Thr Asn Thr Ile Ile Asp Val Gln Ile Ser Thr Pro Arg Pro Val
   225                 230                 235                 240
   Lys Leu Leu Arg Gly His Thr Leu Val Leu Asn Cys Thr Ala Thr Thr
                   245                 250                 255
   Pro Leu Asn Thr Arg Val Gln Met Thr Trp Ser Tyr Pro Asp Glu Lys
               260                 265                 270
   Asn Lys Arg Ala Ser Val Arg Arg Arg Ile Asp Gln Ser Asn Ser His
           275                 280                 285
   Ala Asn Ile Phe Tyr Ser Val Leu Thr Ile Asp Lys Met Gln Asn Lys
       290                 295                 300
   Asp Lys Gly Leu Tyr Thr Cys Arg Val Arg Ser Gly Pro Ser Phe Lys
   305                 310                 315                 320
   Ser Val Asn Thr Ser Val His Ile Tyr Asp Lys Ala Phe Ile Thr Val
                   325                 330                 335
   Lys His Arg Lys Gln Gln Val Leu Glu Thr Val Ala Gly Lys Arg Ser
               340                 345                 350
   Tyr Arg Leu Ser Met Lys Val Lys Ala Phe Pro Ser Pro Glu Val Val
           355                 360                 365
   Trp Leu Lys Asp Gly Leu Pro Ala Thr Glu Lys Ser Ala Arg Tyr Leu
       370                 375                 380
   Thr Arg Gly Tyr Ser Leu Ile Ile Lys Asp Val Thr Glu Glu Asp Ala
   385                 390                 395                 400
   Gly Asn Tyr Thr Ile Leu Leu Ser Ile Lys Gln Ser Asn Val Phe Lys
                   405                 410                 415
   Asn Leu Thr Ala Thr Leu Ile Val Asn Val Lys Pro Gln Ile Tyr Glu
               420                 425                 430
   Lys Ala Val Ser Ser Phe Pro Asp Pro Ala Leu Tyr Pro Leu Gly Ser
           435                 440                 445
   Arg Gln Ile Leu Thr Cys Thr Ala Tyr Gly Ile Pro Gln Pro Thr Ile
       450                 455                 460
   Lys Trp Phe Trp His Pro Cys Asn His Asn His Ser Glu Ala Arg Cys
   465                 470                 475                 480
   Asp Phe Cys Ser Asn Asn Glu Glu Ser Phe Ile Leu Asp Ala Asp Ser
                   485                 490                 495
   Asn Met Gly Asn Arg Ile Glu Ser Ile Thr Gln Arg Met Ala Ile Ile
               500                 505                 510
   Glu Gly Lys Asn Lys Met Ala Ser Thr Leu Val Val Ala Asp Ser Arg
           515                 520                 525
   Ile Ser Gly Ile Tyr Ile Cys Ile Ala Ser Asn Lys Val Gly Thr Val
       530                 535                 540
   Gly Arg Asn Ile Ser Phe Tyr Ile Thr Asp Val Pro Asn Gly Phe His
   545                 550                 555                 560
   Val Asn Leu Glu Lys Met Pro Thr Glu Gly Glu Asp Leu Lys Leu Ser
                   565                 570                 575
   Cys Thr Val Asn Lys Phe Leu Tyr Arg Asp Val Thr Trp Ile Leu Leu
               580                 585                 590
   Arg Thr Val Asn Asn Arg Thr Met His Tyr Ser Ile Ser Lys Gln Lys
           595                 600                 605
   Met Ala Ile Thr Lys Glu His Ser Ile Thr Leu Asn Leu Thr Ile Met
       610                 615                 620
   Asn Val Ser Leu Gln Asp Ser Gly Thr Tyr Ala Cys Arg Ala Arg Asn
   625                 630                 635                 640
   Val Tyr Thr Gly Glu Glu Ile Leu Gln Lys Lys Glu Ile Thr Ile Arg
                   645                 650                 655
   Asp Gln Glu Ala Pro Tyr Leu Leu Arg Asn Leu Ser Asp His Thr Val
               660                 665                 670
   Ala Ile Ser Ser Ser Thr Thr Leu Asp Cys His Ala Asn Gly Val Pro
           675                 680                 685
   Glu Pro Gln Ile Thr Trp Phe Lys Asn Asn His Lys Ile Gln Gln Glu
       690                 695                 700
   Pro Gly Ile Ile Leu Gly Pro Gly Ser Ser Thr Leu Phe Ile Glu Arg
   705                 710                 715                 720
   Val Thr Glu Glu Asp Glu Gly Val Tyr His Cys Lys Ala Thr Asn Gln
                   725                 730                 735
   Lys Gly Ser Val Glu Ser Ser Ala Tyr Leu Thr Val Gln Gly Thr Ser
               740                 745                 750
   Asp Lys Ser Asn Leu Glu Leu Ile Thr Leu Thr Cys Thr Cys Val Ala
           755                 760                 765
   Ala Thr Leu Phe Trp Leu Leu Leu Thr Leu Phe Ile Arg Lys Met Lys
       770                 775                 780
   Arg Ser Ser Ser Glu Ile Lys Thr Asp Tyr Leu Ser Ile Ile Met Asp
   785                 790                 795                 800
   Pro Asp Glu Val Pro Leu Asp Glu Gln Cys Glu Arg Leu Pro Tyr Asp
                   805                 810                 815
   Ala Ser Lys Trp Glu Phe Ala Arg Glu Arg Leu Lys Leu Gly Lys Ser
               820                 825                 830
   Leu Gly Arg Gly Ala Phe Gly Lys Val Val Gln Ala Ser Ala Phe Gly
           835                 840                 845
   Ile Lys Lys Ser Pro Thr Cys Arg Thr Val Ala Val Lys Met Leu Lys
       850                 855                 860
   Glu Gly Ala Thr Ala Ser Glu Tyr Lys Ala Leu Met Thr Glu Leu Lys
   865                 870                 875                 880
   Ile Leu Thr His Ile Gly His His Leu Asn Val Val Asn Leu Leu Gly
                   885                 890                 895
   Ala Cys Thr Lys Gln Gly Gly Pro Leu Met Val Ile Val Glu Tyr Cys
               900                 905                 910
   Lys Tyr Gly Asn Leu Ser Asn Tyr Leu Lys Ser Lys Arg Asp Leu Phe
           915                 920                 925
   Phe Leu Asn Lys Asp Ala Ala Leu His Met Glu Pro Lys Lys Glu Lys
       930                 935                 940
   Met Glu Pro Gly Leu Glu Gln Gly Lys Lys Pro Arg Leu Asp Ser Val
   945                 950                 955                 960
   Thr Ser Ser Glu Ser Phe Ala Ser Ser Gly Phe Gln Glu Asp Lys Ser
                   965                 970                 975
   Leu Ser Asp Val Glu Glu Glu Glu Asp Ser Asp Gly Phe Tyr Lys Glu
               980                 985                 990
   Pro Ile Thr Met Glu Asp Leu Ile  Ser Tyr Ser Phe Gln  Val Ala Arg
           995                 1000                 1005
   Gly Met  Glu Phe Leu Ser Ser  Arg Lys Cys Ile His  Arg Asp Leu
       1010                 1015                 1020
   Ala Ala  Arg Asn Ile Leu Leu  Ser Glu Asn Asn Val  Val Lys Ile
       1025                 1030                 1035
   Cys Asp  Phe Gly Leu Ala Arg  Asp Ile Tyr Lys Asn  Pro Asp Tyr
       1040                 1045                 1050
   Val Arg  Lys Gly Asp Thr Arg  Leu Pro Leu Lys Trp  Met Ala Pro
       1055                 1060                 1065
   Glu Ser  Ile Phe Asp Lys Ile  Tyr Ser Thr Lys Ser  Asp Val Trp
       1070                 1075                 1080
   Ser Tyr  Gly Val Leu Leu Trp  Glu Ile Phe Ser Leu  Gly Gly Ser
       1085                 1090                 1095
   Pro Tyr  Pro Gly Val Gln Met  Asp Glu Asp Phe Cys  Ser Arg Leu
       1100                 1105                 1110
   Arg Glu  Gly Met Arg Met Arg  Ala Pro Glu Tyr Ser  Thr Pro Glu
       1115                 1120                 1125
   Ile Tyr  Gln Ile Met Leu Asp  Cys Trp His Arg Asp  Pro Lys Glu
       1130                 1135                 1140
   Arg Pro  Arg Phe Ala Glu Leu  Val Glu Lys Leu Gly  Asp Leu Leu
   1145 1150 1155
   Gln Ala Asn Val Gln Gln Asp Gly Lys Asp Tyr Ile Pro Ile Asn
   1160 1165 1170
   Ala Ile Leu Thr Gly Asn Ser Gly Phe Thr Tyr Ser Thr Pro Ala
   1175 1180 1185
   Phe Ser Glu Asp Phe Phe Lys Glu Ser Ile Ser Ala Pro Lys Phe
   1190 1195 1200
   Asn Ser Gly Ser Ser Asp Asp Val Arg Tyr Val Asn Ala Phe Lys
   1205 1210 1215
   Phe Met Ser Leu Glu Arg Ile Lys Thr Phe Glu Glu Leu Leu Pro
   1220 1225 1230
   Asn Ala Thr Ser Met Phe Asp Asp Tyr Gln Gly Asp Ser Ser Thr
   1235 1240 1245
   Leu Leu Ala Ser Pro Met Leu Lys Arg Phe Thr Trp Thr Asp Ser
   1250 1255 1260
   Lys Pro Lys Ala Ser Leu Lys Ile Asp Leu Arg Val Thr Ser Lys
   1265 1270 1275
   Ser Lys Glu Ser Gly Leu Ser Asp Val Ser Arg Pro Ser Phe Cys
   1280 1285 1290
   His Ser Ser Cys Gly His Val Ser Glu Gly Lys Arg Arg Phe Thr
   1295 1300 1305
   Tyr Asp His Ala Glu Leu Glu Arg Lys Ile Ala Cys Cys Ser Pro
   1310 1315 1320
   Pro Pro Asp Tyr Asn Ser Val Val Leu Tyr Ser Thr Pro Pro Ile
   1325 1330 1335
<210> 11
   <211> 117
   <212> PRT
   <213> homo sapiens
<220>
   <221> MISC_FEATURE
   <223> relaxine
<400> 11
   Met Pro Arg Leu Phe Phe Phe His Leu Leu Gly Val Cys Leu Leu Leu
   1               5                   10                  15
   Asn Gln Phe Ser Arg Ala Val Ala Asp Ser Trp Met Glu Glu Val Ile
               20                  25                  30
   Lys Leu Cys Gly Arg Glu Leu Val Arg Ala Gln Ile Ala Ile Cys Gly
           35                  40                  45
   Met Ser Thr Trp Ser Lys Arg Ser Leu Ser Gln Glu Asp Ala Pro Gln
       50                  55                  60
   Thr Pro Arg Pro Val Ala Gly Asp Phe Ile Gln Thr Val Ser Leu Gly
   65                  70                  75                  80
   Ile Ser Pro Asp Gly Gly Lys Ala Leu Arg Thr Gly Ser Cys Phe Thr
                   85                  90                  95
   Arg Glu Phe Leu Gly Ala Leu Ser Lys Leu Cys His Pro Ser Ser Thr
               100                 105                 110
   Lys Ile Gln Lys Pro
           115

## Description

### RESUME

Les présents inventeurs ont identifié des biomarqueurs plasmatiques qui, seuls ou en combinaison, permettent de diagnostiquer une cardiomyopathie du peripartum (CMPP) chez une patiente de façon certaine. Le dosage de ces biomarqueurs peut se faire facilement à partir d'une gouttelette de sang, par exemple prélevée au bout du doigt d'une patiente, ou de plasma sanguin. Ces biomarqueurs sont des molécules connues pour leur effet pro- ou anti-angiogénique, plus précisément la fms like tyrosine kinase 1 (sFlt-1), le P1GF, VEGF et la relaxine.

### DESCRIPTION DE L'ART ANTERIEUR

La cardiomyopathie du peripartum (CMPP) est une insuffisance cardiaque aiguë, survenant dans le dernier mois d'une grossesse, ou dans les 6 mois qui suivent l'accouchement, chez une femme n'ayant préalablement aucun problème cardiaque. La CMPP a une incidence d'environ un cas pour 3 000 grossesses aux Etats-Unis, avec un risque doublé chez les femmes noires ; l'incidence semble très supérieure en Afrique du Sud (1/1000 grossesses) et à Haïti (de l'ordre d'un cas pour 300 grossesses) (Patten IS et al, Nature 2012). En revanche, l'incidence de cette maladie en Europe n'est pas connue.

La gravité de cette maladie réside dans le fait que les patientes peuvent se présenter soit pour dyspnée (symptôme fréquent en cours de grossesse), soit dans un tableau d'insuffisance cardiaque (difficile à diagnostiquer en fin de grossesse) ou plus rarement dans un tableau de choc cardiogénique. Le pronostic est variable avec une récupération sans rechute dans 23% des cas, une insuffisance cardiaque chronique dans 50% des cas et un décès dans 15% des cas (Sliwa K et al, Lancet 2006).

Les complications de la CMPP sont fréquentes et graves : la persistance de la dysfonction cardiaque touche au moins un tiers des CMPP et la mortalité est proche de 5-10% dans les mois qui suivent la découverte de la maladie. De plus, des données montrent que la mortalité est encore plus grande pour les femmes ayant présenté une CMPP, lors de la grossesse suivante.

Il a récemment été proposé que cette maladie serait liée à la production d'une prolactine modifiée par les femmes allaitantes, car cette prolactine modifiée aurait un effet négatif sur le cœur (Hiliker-Kleiner D. et al, Cell 2007). Un article récent (Patten IS et al. Nature 2012) évoque par ailleurs un lien entre l'excès d'anti-angiogénèse et la dysfonction cardiaque de la CMPP. Cet article suggère que la CMPP serait causée par une élévation du taux circulant de sFlt-1 (facteur anti-angiogénique). Ces résultats ne sont pas fiables en raison des trois critiques majeures: 1) les patientes incluses dans cette étude souffraient d'une forte incidence de pré-éclampsie ; 2) la comparaison de sFlt-1 plasmatique s'est faite versus des patients contrôles, a priori pas dans la phase du péripartum et 3) seul le sFlt-1 a été dosé et pas l'ensemble de la balance angiogénique/antiangiogénique. Par ailleurs, la demande internationale WO 2012/106152 suggère qu'un taux élevé de facteurs anti-angiogéniques (et notamment le sFlt-1) dans le plasma sanguin est le signe d'un risque accru de développer une maladie cardiaque chronique comme la CMPP.

A l'inverse, les présents inventeurs ont mis en évidence que l'apparition de la CMPP résulte en fait d'une persistance anormale de l'angiogenèse et d'un effondrement de la relaxine et du sFlt-1 à la fin de la grossesse et après l'accouchement. En effet, la grossesse nécessite un développement massif de la circulation sanguine de la mère pour pouvoir nourrir le fœtus. A la fin de la grossesse, un agent anti-angiogénique (sFlt-1) est sécrété pour que le système vasculaire revienne à l'état pré-grossesse. Les résultats des inventeurs démontrent que la CMPP n'est pas liée, comme le suggérait les précédentes études (Patten IS et WO 2012/106152), à une élévation du taux de facteurs anti-angiogéniques circulants, mais elle est associée au contraire à des taux effondrés d'agents anti-angiogéniques (sFlt-1) et à la persistance d'une angiogénèse très active (taux plasmatiques élevés de PIGF et de VEGF) qui se poursuit des mois, malgré l'arrêt de la grossesse. De plus le taux de relaxine est effondré, voire indétectable chez les patientes souffrant de CMPP.

Aujourd'hui, le diagnostic de la CMPP est un diagnostic clinique et échocardiographique d'exclusion, ce qui le rend peu fiable et couteux (Sliwa K et al, Lancet 2006). De plus, les échocardiographistes sont totalement absents des maternités voire des régions dans le monde où la CMPP est endémique. Enfin, il n'existe aucun biomarqueur diagnostic fiable pour détecter cette maladie. Dans ce contexte, les présents inventeurs ont cherché à identifier des biomarqueurs plasmatiques révélateurs de cette maladie, dont la mesure serait aisée dans des laboratoires de biologie (biomarqueurs dits « diagnostiques »).

### DESCRIPTION DETAILLEE DE L'INVENTION

Dans ce but, les inventeurs ont étudié les concentrations plasmatiques de facteurs angiogéniques (pro et anti) et de relaxine dans des prélèvements de patientes saines dans la phase du péripartum (patientes enceintes ou après leur accouchement, toutes sans CMPP) et l'ont comparée aux taux mesurés chez des patients admises dans les hôpitaux pour CMPP.

Les inventeurs ont alors pu confirmer que - contrairement à ce qui était décrit dans la littérature scientifique - la CMPP survient pendant ou après la grossesse 1) lorsque l'angiogénèse persiste de façon anormale (au lieu de diminuer), et que cette persistance est attestée par des niveaux anormalement élevés de facteurs pro-angiogéniques (tels que le VEGF et le PlGF),des niveaux anormalement faibles de facteurs anti-angiogéniques (tel que le sFlt-1) et 2) quand le taux circulant de relaxine, est effondré (il est même indétectable chez beaucoup de patientes souffrant de CMPP), ce qui induit des ratios [sFlt-1/PlGF], [sFlt-1/VEGF], [Relaxine/PlGF] et/ou [Relaxine/VEGF] mesurés anormalement bas.

### Description des biomarqueurs de l'invention

Dans un premier aspect, la présente invention vise une méthode pour diagnostiquer une cardiomyopathie peripartum (CMPP) chez une femme non pré-éclamptique, ladite méthode comprenant les étapes de:
a) mesurer *in vitro,* dans un échantillon de sang de ladite femme, le taux de VEGF, le PIGF, le sFlt-1 et/ou la relaxine,
b) comparer ce(s) taux à une (des) valeur(s) de référence,
dans laquelle si le taux de PlGF mesuré à l'étape a) est supérieur à la valeur de référence pour ce marqueur, et/ou si le taux de VEGF est supérieur à la valeur de référence pour ce marqueur, et/ou si le taux de sFlt-1 mesuré à l'étape a) est inférieur à la valeur de référence pour ce marqueur, et/ou si le taux de relaxine mesuré à l'étape a) pour ce marqueur est supérieur à la valeur de référence pour ce marqueur, alors cette femme souffre d'une CMPP.

Cette méthode est caractérisée en ce que, si le taux de facteur pro-angiogénique VEGF et/ou PlGF mesuré à l'étape a) est supérieur à ladite valeur de référence et/ou si le taux de facteur anti-angiogénique sFlt-1 et/ou de relaxine mesurés à l'étape a) est inférieur à ladite valeur de référence, alors ladite femme souffre d'une CMPP.

Par « facteurs modulant l'angiogénèse », sont désignés dans la présente demande tous les facteurs pro-angiogéniques et anti-angiogéniques aujourd'hui connus, ainsi que tout autre facteur dont le rôle (activateur ou inhibiteur) sur l'angiogénèse viendrait à être révélé dans les années futures. La relaxine fait partie de ces facteurs. Son activité pro-angiogénique, décrite dans l'art antérieur (notamment dans Dschietzig T, Pharmacol Ther. 2006), est en effet aujourd'hui remise en question par les résultats récemment obtenus par les présents inventeurs (cf. exemples ci-dessous). Si la relaxine module bien l'angiogénèse, sa nature pro- ou anti-angiogénique nécéssite donc aujourd'hui confirmation.

Lesdits facteurs pro-angiogéniques sont choisis dans le groupe constitué par : PlGF et VEGF.

Ledit facteur anti-angiogénique est le sFlt-1.

Lesdits facteurs modulant l'angiogénèse sont choisis parmi les facteurs pro-angiogéniques tels que le VEGF ou le PlGF et les facteurs anti-angiogéniques tel que le sFlt-1. Ledit facteur modulant l'angiogénèse est la relaxine.

Le « facteur de croissance placentaire humain » ou **«PlGF»** (pour « Placental Growth factor ») ou « PGF » est une protéine qui est codée par le gène *PGF.* Cette protéine est membre de la sous-famille du VEGF (Vascular Endothelial Growth factor), qui est impliquée dans l'angiogénèse et la vasculogénèse, notamment pendant l'embryogénèse. La principale source de PlGF pendant la grossesse est le trophoblaste placentaire. Chez l'être humain, cette protéine a pour séquence SEQ ID NO :1 (NP_001193941). Le dosage du PlGF circulant peut être effectué par n'importe quelle méthode classique connue de l'homme du métier. En particulier, il est possible d'obtenir une valeur très précise du taux de PlGF circulant en utilisant les tests commercialisés à cet effet (Roche, PerlinElmer). Ces tests sont classiquement utilisés pour diagnostiquer une pré-éclampsie chez les femmes enceintes.

Le **VEGF** est protéine de signalisation importante impliquée à la fois dans la vasculogenèse (la formation *de novo* de l'appareil circulatoire embryonnaire) et l'angiogenèse (la croissance des vaisseaux sanguins à partir de vaisseaux préexistants). Le VEGF a, chez l'homme, plusieurs isoformes. Ces isoformes ont pour séquence NP_001020537 (isoforme A, SEQ ID NO :2), NP_003367.4 (isoforme B, SEQ ID NO :3), NP_001020538.2 (isoforme C, SEQ ID NO :4), NP_001020539.2 (isoforme D, SEQ ID NO :5), NP_001020540 (isoforme E, SEQ ID NO :6), etc. Ces isoformes du VEGF peuvent être dosés par n'importe quelle méthode classique connue de l'homme du métier, notamment par ELISA, par exemple au moyen de kits commercialisés (par exemple le kit ADI-900-080 chez Enzo Life Sciences ou 45-VEGHU-E01 chez Alpco immunoassays).

La « tyrosine kinase-1 fms-like soluble » ou « **sFlt-1** » (pour « Soluble fms-like tyrosine kinase-1 ») ou « sVEGFR-1 » est une protéine tyrosine kinase anti-angiogénique, i.e., qui inhibe les protéines impliquées dans la croissance des vaisseaux sanguins. Cette kinase est un variant d'épissage du récepteur 1 de VEGF (Flt-1) qui est produite par divers tissus (Khalil A. et al, PLoS One 2008). Elle agit comme un récepteur du facteur de croissance endothélial vasculaire (VEGF) et réduit les taux circulants libres des facteurs pro-angiogéniques VEGF et PlGF. Les taux sériques de sFlt-1 sont élevés chez les femmes atteintes de pré-éclampsie avant même la présentation clinique et sFlt-1 serait associé à la gravité de cette maladie et à l'apparition de ses symptômes (Troisi R. et al, Am. J. Obstet. Gynecol. 2008). sFlt-1 baisse les concentrations plasmatiques et émousse ainsi les effets bénéfiques de ces facteurs pro-angiogéniques sur l'endothélium maternel ce qui générerait une hypertension maternelle importante et une protéinurie. En cas de grossesse normale, les niveaux du facteur anti-angiogénique sFlt-1 restent stables au cours des stades précoces et en milieu de gestation et augmentent progressivement jusqu'à terme. Chez les femmes qui développent une pré-eclampsie, les niveaux plasmatiques de sFlt-1 sont plus élevés que dans la grossesse normale (Maynard SE et al, J. Clin. Invest. 2003; Levine R.J., JAMA 2005; Lam C., Hypertension 2005) et le niveau plasmatique de PlGF est anormalement bas. Le rapport sFlt-1/PlGF est actuellement utilisé pour diagnostiquer la pré-éclampsie et prédire le moment de l'accouchement. Un rapport sFlt-1/PlGF > 85 indique une pré-éclampsie (Rana et al. Circulation. 2012;125:911-919). Le sFlt-1 a, chez l'homme, la séquence SEQ ID NO : 7 (NP_001153503.1, isoforme 4), SEQ ID NO :8 (NP_001153502.1,isoforme 3), SEQ ID NO :9 (NP_001153392.1, isoforme 2) ou SEQ ID NO :10 (NP_002010.2, isoforme 1). Le dosage du sFlt-1 circulant peut être effectué par n'importe quelle méthode classique connue de l'homme du métier. En particulier, il est possible d'obtenir une valeur très précise du taux de sFlt-1 circulant en utilisant les tests commercialisés à cet effet (Roche). Toutes les isoformes de sFlt-1 sont reconnues par les anticorps distribués dans le commerce.

La **relaxine** est une hormone polypeptidique de structure hétérodimérique appartenant à la superfamille de l'insuline qui contient aussi IGF-I et IGF-II (Insuline-like growth factors I et II) et RLF ou INSL3 (Relaxine-like factor) (Chartrel N. et al, M/S: médecine sciences 2002). Il existe chez l'homme, 3 isoformes de relaxine, d'action et de cibles différentes : les relaxine 1, 2 et 3. Seule la relaxine 2 est retrouvée sous forme circulante chez l'homme et serait la seule à avoir une action hormonale (Bathgate RA et al, J. Biol. Chem. 2002). Elle a pour séquence EAW58771.1 (SEQ ID NO :11). Ce facteur pro-angiogénique est impliqué dans divers phénomènes physiologiques tels que la régulation du tonus vasomoteur, l'osmolalité plasmatique, l'angiogenèse, la clairance du collagène et la fonction rénale (Dschietzig T, Pharmacol. Ther. 2006). Son effet thérapeutique a été testé très récemment au cours de l'insuffisance cardiaque aigue sans choc cardiogénique avec des résultats positifs très encourageants (Teerlink JR et al, Lancet 2009). Il a été montré qu'une dose de relaxine de 30 µg/kg par jour améliorait la dyspnée d'une manière significative à partir de la sixième heure après son administration et qu'elle avait un effet rémanent puisque cette amélioration se prolongeait jusqu'au 14^{ème} jour. Le traitement par la relaxine permet aussi l'amélioration des signes d'insuffisance cardiaque, raccourcit la durée d'hospitalisation, réduit l'aggravation des symptômes au cours de l'hospitalisation, diminue le risque de décès de cause cardiovasculaire ou de réadmission à deux mois de suivi et enfin diminue la mortalité cardiovasculaire à trois mois (Teerlink JR et al, Lancet 2009)*.* La relaxine joue un rôle majeur dans le processus de reproduction chez les mammifères comme le porc, le cobaye et le rat puisqu'il a été prouvé que sa sécrétion augmente et qu'elle agit directement sur la croissance et le remodelage des organes génitaux pendant la gestation et au moment de la partition en provoquant l'élongation des ligaments pelviens et le relâchement du col utérin (Sherwood OD, Endocr. Rev. 2004, April 25(2):205-34).

Chez la femme, aucune des propriétés physiologiques de la relaxine n'a été confirmée, mais il a été montré que la relaxine jouerait un rôle lors de l'induction de la décidualisation. La relaxine est détectée dans le sang chez la femme pendant la phase lutéale à 6 pg/ml et augmente rapidement en cas de fécondation atteignant le pic de 1ng/ml à la fin du premier trimestre, puis baisse et se maintient à 0,5 ng/ml jusqu'au terme. Chez la femme ménopausée, les taux circulants de relaxine sont effondrés (Sherwood OD, Endocr. Rev. 2004: April 25(2):205-34).

La concentration plasmatique de relaxine a été décrite comme augmentée chez l'insuffisant cardiaque, en fonction de la sévérité (Dschietzig T et al FASEB 15 :2187-2195, 2001). La relaxine peut être dosée par n'importe quelle méthode classique connue de l'homme du métier, par exemple par ELISA.

Les résultats des inventeurs montrent pour la première fois que, si le taux de PlGF plasmatique est élevé (par exemple supérieur à 60 pg/ml), ou si te taux de VEGF est élevé (par exemple supérieur à 120 pg/ml), ou si le taux de sFlt-1 plasmatique est faible (par exemple inférieur à 480 pg/ml), ou si le taux de relaxine plasmatique est faible (par exemple inférieur à 10 pg/mL) chez une femme dans les suites d'un accouchement, le diagnostic de CMPP est certain. Les inventeurs proposent donc de diagnostiquer la CMPP chez une patiente en dosant la quantité de PlGF, de sFlt-1, de VEGF ou de relaxine dans un échantillon biologique de ladite femme. Les quatre biomarqueurs identifiés par les présents inventeurs (à savoir le P1GF, le sFlt-1, le VEGF et la relaxine) peuvent être utilisés indépendamment les uns des autres. Ils seront par la suite qualifiés de « biomarqueurs diagnostiques de l'invention ». Ces biomarqueurs englobent également les ratios [relaxine/PlGF], [relaxine/VEGF], [sFlt-1/PlGF] et [sFlt-1/VEGF]).

La présente demande vise donc une méthode pour diagnostiquer une cardiomyopathie peripartum (CMPP) chez une femme non pré-éclamptique, ladite méthode comprenant les étapes de:
a) mesurer *in vitro,* dans un échantillon de sang de ladite femme, le taux de sFlt-1, de P1GF, de VEGF et/ou de relaxine,
b) comparer ces taux à des valeurs de référence,
dans laquelle si le taux de PlGF mesuré à l'étape a) est supérieur à la valeur de référence pour ce marqueur, et/ou si le taux de VEGF est supérieur à la valeur de référence pour ce marqueur, et/ou si le taux de sFlt-1 mesuré à l'étape a) est inférieur à la valeur de référence pour ce marqueur, et/ou si le taux de relaxine mesuré à l'étape a) pour ce marqueur est supérieur à la valeur de référence pour ce marqueur, alors cette femme souffre d'une CMPP.

Dans un mode de réalisation plus particulier, la présente demande vise une méthode comprenant les étapes :
a) mesurer *in vitro* le taux de VEGF et/ou de PlGF dans un échantillon de sang de cette femme,
b) comparer ce(s) taux à une (des) valeur(s) de référence,
dans laquelle, un taux de VEGF et/ou de PlGF mesuré à l'étape a) supérieur à ladite valeur de référence indique que cette femme souffre d'une CMPP.

Dans un autre mode de réalisation plus particulier, la présente demande vise une méthode comprenant les étapes :
a) mesurer *in vitro* le taux de sFlt-1 dans un échantillon biologique de cette femme,
b) comparer ce(s) taux à une (des) valeur(s) de référence,
dans laquelle un taux de sFlt-1 mesuré à l'étape a) inférieur à ladite valeur de référence indique que cette femme souffre d'une CMPP.

Le dosage du sFlt-1 et du PlGF circulant peut être effectué par n'importe quelle méthode classique connue de l'homme du métier. En particulier, il est possible d'obtenir une valeur très précise du taux de ces marqueurs en utilisant les tests commercialisés à cet effet (Roche). Ces tests sont classiquement utilisés pour diagnostiquer une pré-éclampsie chez les femmes enceintes. De même, le dosage du VEGF et du PlGF peut être réalisé par n'importe quelle méthode (par ELISA par exemple).

Par « diagnostiquer une CMPP», on entend ici détecter si une femme est en train de développer ou a déjà développé une CMPP au moment où l'échantillon biologique est prélevé. De préférence, cette femme ne souffre pas d'éclampsie ni de pré-éclampsie.

Par « CMPP » ou « cardiomyopathie du péri-partum », on entend ici désigner un tableau d'insuffisance cardiaque survenant typiquement en fin de grossesse et jusqu'à six mois après l'accouchement. La cardiomyopathie se manifeste par un tableau d'insuffisance cardiaque sans particularité : essoufflement, œdèmes des membres inférieurs. L'échocardiographie montre une dilatation plus ou moins importante du ventricule gauche avec une fraction d'éjection basse, signant la dysfonction systolique. Le taux de BNP ou de NT-proBNP dans le sang est élevé, comme dans toute insuffisance cardiaque. Il semble qu'un taux élevé de prolactine et d'interféron gamma soit corrélé avec une forme plus grave.

La « valeur de référence du biomarqueur PlGF » en ce qui concerne le diagnostic de la CMPP est typiquement d'environ 60 pg/mL. De préférence, elle est de 80 pg/mL et, de manière encore plus préférée, de 90 pg/mL.

D'autre part, la « valeur de référence du biomarqueur VEGF» en ce qui concerne le diagnostic de la CMPP est typiquement d'environ 120 pg/mL. De préférence, elle est d'environ 130 pg/mL et, de manière encore plus préférée, de 150 pg/mL.

Dans le contexte de la présente invention, le taux de PlGF ou de VEGF dosé est dit « supérieur à la valeur de référence», si le ratio entre ce taux et ladite valeur de référence est supérieur à 1.4, de préférence supérieur à 1.6, de manière plus préférée supérieur à 1.8.

Par ailleurs, la « valeur de référence pour le sFlt-1 » en ce qui concerne le diagnostic de la CMPP est typiquement d'environ 480 pg/mL. De préférence, elle est d'environ 300 pg/mL et, de manière encore plus préférée, de 200 pg/mL.

Dans un autre mode de réalisation préféré entre tous, la présente demande vise une méthode comprenant les étapes :
a) mesurer *in vitro* le taux de relaxine dans un échantillon de sang de cette femme,
b) comparer ce taux à une valeur de référence,
dans laquelle un taux de relaxine mesuré à l'étape a) inférieur à ladite valeur de référence indique que cette femme souffre d'une CMPP.

De façon intéressante, il sera possible de traiter les patientes diagnostiquées comme souffrant d'une CMPP par cette méthode en leur administrant de la relaxine recombinante, tel que décrit dans l'insuffisance cardiaque aiguë hors du contexte de la grossesse (Teerlink et al. Lancet 2012). Un tel traitement sera en effet susceptible de rétablir la concentration de relaxine ; on peut donc imaginer adapter la posologie au taux plasmatique de relaxine initial et de répéter l'administration de la relaxine recombinante jusqu'à obtenir le taux plasmatique de relaxine adapté. Le rétablissement de taux de relaxine plasmatique peut possiblement rétablir l'équilibre des facteurs pro- et anti-angiogéniques chez la patiente, afin d'améliorer les fonctions cardiovasculaires et la fonction des organes tels que le rein et le foie (Metra et al. JACC 2013).

De manière plus générale, il sera possible d'administrer aux patientes diagnostiquées comme souffrant de CMPP selon l'une quelconque des méthodes de l'invention (basée sur la mesure de sFlt-1, PlGF, VEGF et/ou de relaxine et/ou de leur ratio respectif) en administrant de la relaxine recombinante (ou serelaxine) ou de la bromocriptine.

La méthode de l'invention pourra alors avantageusement faire partie d'une méthode de traitement visant à soulager ces patientes.

Dans ce cas particulier, la présente description vise également des méthodes de traitement contenant i) l'une quelconque des méthodes diagnostiques de l'invention (basée sur la mesure de sFlt-1, PlGF, VEGF et/ou de relaxine et/ou de leur ratio respectif), et ii) l'administration de traitements connus pour soulager les symptômes de cette maladie, notamment de la relaxine recombinante (ou serelaxine) ou la bromocriptine ou du sFlt-1 recombinant. D'autres traitements sont décrits notamment dans Sliwa K et al, European Journal of Heart Failure, 2010 et dans Dickstein K. et al, European Journal of Heart Failure, 2008. Il s'agit par exemple d'administrer de l'oxygène, du nitrate, des agents ionotropes (dobutamine ou levosimendan) ou d'effectuer une transplantation cardiaque ou d'administrer des traitements oraux au long cours à base d'inhibiteurs d'enzyme de conversion, antagonistes des récepteurs de l'angiotensine II, de béta-bloqueurs, nitrates, hydralazine, diurétiques, d'antagonistes de l'aldostérone et/ou d'antithrombotiques.

Idéalement, ces méthodes visent à restaurer chez les patientes un taux nominal des biomarqueurs de l'invention. Par exemple, les traitements seront choisis et optimisés (en termes de doses, de fréquence, etc.) afin que lesdits taux atteignent les valeurs de référence recensées dans la présente demande pour chacun de ces biomarqueurs.

Il est donc possible d'utiliser les biomarqueurs de l'invention comme outil de médecine personnalisé.

Les biomarqueurs de l'invention, et notamment le sFlt-1 ou la relaxine, représentent donc des outils de diagnostic compagnons prometteurs, permettant d'évaluer si et dans quelle mesure un traitement à base de relaxine recombinante serait susceptible d'être efficace chez des femmes présentant les symptômes d'une CMPP.

La mesure des biomarqueurs de l'invention permet également d'évaluer l'efficacité d'un traitement et/ou de l'adapter si celui-ci s'avère insuffisant. Dans ce cas, la présente description vise des méthodes de suivi de traitements, comprenant l'étape de mesurer les taux des biomarqueurs de l'invention (sFlt-1, PlGF, VEGF et/ou relaxine ou de leur ratio respectif) avant et après l'administration du traitement, et de comparer ceux-ci. Par exemple, si le taux de sFlt-1 et/ou de relaxine diminue après l'administration d'un traitement, alors celui-ci n'est pas efficace. Il doit être changé ou sa dose doit être augmentée. En outre, si le taux de PlGF ou de VEGF augmente après l'administration du traitement, alors celui-ci n'est pas efficace. Il doit être changé ou sa dose doit être augmentée. A l'inverse, si le taux de sFlt-1 et/ou de relaxine augmente après l'administration d'un traitement, alors ce traitement est efficace et peut être maintenu. En outre, si le taux de PlGF ou de VEGF diminue après l'administration du traitement, alors ce traitement est efficace et peut être maintenu.

La « valeur de référence pour la relaxine » en ce qui concerne le diagnostic de la CMPP est typiquement d'environ 10 pg/mL. De préférence, elle est d'environ 5 pg/mL et, de manière encore plus préférée, de 2 pg/mL.

Dans le contexte de la présente invention, le taux de sFlt-1 ou de relaxine dosé est dit « inférieur à la valeur de référence» si le ratio entre ce taux et ladite valeur de référence est inférieur à 1, de préférence inférieur à 0.8, de manière plus préférée inférieur à 0.6.

De façon intéressante, le dosage de ces biomarqueurs peut se faire facilement à partir d'une gouttelette de sang, par exemple prélevée au bout du doigt d'une patiente. Par « échantillon biologique », on entend donc ici un échantillon de sang, et, plus particulièrement, un échantillon de sérum ou de plasma. Il est également possible de mettre en œuvre la méthode de l'invention à partir d'un échantillon d'urine.

Dans un mode de réalisation particulier, les biomarqueurs diagnostiques de l'invention peuvent être combinés les uns avec les autres. Dans le cadre de la méthode de l'invention, il est possible de mesurer le taux de n'importe quelle combinaison de ces marqueurs : PlGF et VEGF, PlGF et relaxine, PlGF et sFlt-1, sFlt-1 et relaxine, VEGF et sFlt-1, ou encore VEGF et relaxine. Une fois le taux de chaque marqueur mesuré, il est possible de calculer le ratio entre ces taux, afin de poser un diagnostic certain quant à la CMPP chez la femme à qui l'échantillon biologique a été prélevé.

En effet, les résultats des inventeurs montrent pour la première fois que, chez les femmes souffrant de CMPP,
- le ratio entre les taux de relaxine et de PlGF plasmatiques mesurés (ci-après décrit en tant que ratio [relaxine/PlGF]) est proche de zéro (0),
- le ratio entre les taux de relaxine et de VEGF plasmatiques mesurés (ci-après décrit en tant que ratio [relaxine/VEGF]) est proche de zéro (0) ;
- le ratio entre les taux de sFlt-1 et de PlGF plasmatiques mesurés (ci-après décrit en tant que ratio [sFlt-1/PlGF]) est très faible ;
- le ratio entre les taux de sFlt-1 et de VEGF plasmatiques mesurés (ci-après décrit en tant que ratio [sFlt-1/VEGF]) est très faible ;

La mesure de ces ratios permet donc de diagnostiquer de façon certaine la maladie CMPP chez des femmes en fin de grossesse ou à la suite de leur accouchement..

Les inventeurs proposent donc de diagnostiquer la CMPP chez une patiente en dosant la quantité de VEGF ou de PlGF d'une part (facteurs pro-angiogéniques dont le taux est anormalement haut en cas de CMPP), et le taux de sFlt-1 (facteur anti-angiogénique dont le taux est anormalement bas en cas de CMPP) ou de relaxine d'autre part (facteur modulant l'angiogénèse dont le taux est anormalement bas en cas de CMPP). Les quatre combinaisons préférées sont donc : PlGF et relaxine, PlGF et sFlt-1, VEGF et sFlt-1, ou encore VEGF et relaxine.

Ainsi, la méthode diagnostique selon l'invention pourrait contenir les étapes :
a) mesurer le taux de relaxine et le taux de VEGF dans un échantillon de sang de ladite femme, et
b) calculer le ratio [relaxine/VEGF] et le comparer à une valeur de référence
dans laquelle, si le ratio calculé à l'étape b) est inférieur à la valeur de référence pour ce ratio, ou si ce ratio est nul ou quasiment nul (i.e., inférieur à 0.01), alors cette femme souffre d'une CMPP.

La « valeur de référence pour le ratio [relaxine/VEGF] » en ce qui concerne le diagnostic de la CMPP est typiquement d'environ 0.2, et, de manière encore plus préférée, d'environ 0.1. Dans le contexte de la présente invention, le ratio [relaxine/VEGF] est dit « inférieur à la valeur de référence» si le ratio entre le ratio [relaxine/VEGF] et ladite valeur de référence est inférieur à 0.8, de préférence inférieur à 0.6, de manière plus préférée inférieur à 0.4.

Dans un autre mode de réalisation, la méthode diagnostique selon l'invention pourrait contenir les étapes :
a) mesurer le taux de sFlt-1 et le taux de VEGF dans un échantillon de sang de ladite femme, et
b) calculer le ratio [sFlt-1/VEGF] et le comparer à une valeur de référencedans laquelle, si le ratio calculé à l'étape b) est inférieur à la valeur de référence pour ce ratio, alors cette femme souffre d'une CMPP.

La « valeur de référence pour le ratio [sFlt-1/VEGF] » en ce qui concerne le diagnostic de la CMPP est typiquement d'environ 2, et, de manière encore plus préférée, d'environ 1.

Dans le contexte de la présente invention, le ratio [sFlt-1/VEGF] est dit « inférieur à la valeur de référence» si le ratio entre le ratio [sFlt-1/VEGF] et ladite valeur de référence est inférieur à 0.8, de préférence inférieur à 0.6, de manière plus préférée inférieur à 0.4.

Dans un autre mode de réalisation, la méthode diagnostique selon l'invention pourrait contenir les étapes :
a) mesurer le taux de sFlt-1 et le taux de PlGF dans un échantillon de sang de ladite femme, et
b) calculer le ratio [sFlt-1/PlGF] et le comparer à une valeur de référence,dans laquelle, si le ratio calculé à l'étape b) est inférieur à la valeur de référence pour ce ratio, alors cette femme souffre d'une CMPP.

La « valeur de référence pour le ratio [sFlt-1/PlGF] » en ce qui concerne le diagnostic de la CMPP est typiquement d'environ 5, et, de manière encore plus préférée, d'environ 3.

Dans le contexte de la présente invention, le ratio [sFlt-1/PlGF] est dit « inférieur à la valeur de référence» si le ratio entre le ratio [sFlt-1/PlGF] et ladite valeur de référence est inférieur à 0.8, de préférence inférieur à 0.6, de manière plus préférée inférieur à 0.4.

Dans un autre mode de réalisation, la méthode diagnostique selon l'invention pourrait contenir les étapes :
a) mesurer le taux de relaxine et le taux de PlGF dans un échantillon de sang de ladite femme, et
b) calculer le ratio [relaxine /PlGF] et le comparer à une valeur de référence, dans laquelle si le ratio calculé à l'étape b) est inférieur à la valeur de référence pour ce ratio ou si ce ratio est nul ou quasiment nul (i.e., inférieur à 0.01), alors cette femme souffre d'une CMPP.

La « valeur de référence pour le ratio [relaxine/PlGF] » en ce qui concerne le diagnostic de la CMPP est typiquement d'environ 0.2, et, de manière encore plus préférée, d'environ 0.1.

Dans le contexte de la présente invention, le ratio [relaxine/PlGF] est dit « inférieur à la valeur de référence» si le ratio entre le ratio [relaxine/PlGF] et ladite valeur de référence est inférieur à 0.8, de préférence inférieur à 0.6, de manière plus préférée inférieur à 0.4.

Il est possible, pour réaliser ces méthodes diagnostiques, de doser les différents marqueurs dans un seul et même échantillon ou dans différents échantillons prélevés chez la même patiente. Dans ce deuxième cas, les prélèvements ne doivent pas avoir été espacés dans le temps de plus de 1 jour (il est même préférable de les prélever à moins d'une heure d'écart).

La méthode diagnostique de l'invention est destinée à détecter la CMPP chez n'importe quelle femme en âge de procréer (typiquement, âgée de 16 à 55 ans). Les résultats des inventeurs montrent par ailleurs que l'origine de la patiente (caucasienne, africaine, etc.) n'a pas d'importance. Cependant, il est important que la patiente ne souffre pas d'éclampsie ni de pré-éclampsie (cette maladie n'est en fait pas liée à la CMPP).

La CMPP étant une maladie survenant en fin ou après une grossesse, cette méthode est plus particulièrement destinée à diagnostiquer la CMPP chez une femme qui est enceinte au moment où l'échantillon a été prélevé, ou chez une femme qui a accouché dans les six mois précédent le prélèvement de l'échantillon. De manière encore plus préférée, cette méthode est destinée aux femmes en fin de grossesse (typiquement, dans le troisième trimestre de grossesse) au moment où l'échantillon a été prélevé, ou ayant accouché dans les mois (notamment dans les six mois) précédent le prélèvement du (ou des)échantillon(s).

Dans un second aspect, la présente invention vise également des kits contenant les moyens de détecter et/ou de quantifier les biomarqueurs pronostics et diagnostiques de l'invention. Ces kits permettent d'évaluer si une patiente souffre d'une CMPP, afin de lui procurer un traitement adapté.

Ces kits peuvent contenir des anticorps pour détecter le PlGF, le sFlt-1, le VEGF et/ou la relaxine, dans un échantillon biologique tel qu'un échantillon sanguin.

Ce kit contient des moyens de détection et/ou de mesure i) du taux de PlGF et de relaxine, ii) du taux de VEGF et de relaxine, iii) du taux de PlGF et de sFlt-1 ou iv) du taux de sFlt-1 et de VEGF, dans un échantillon biologique tel qu'un échantillon sanguin. En particulier, la présente invention concerne :
- un kit comprenant au moins un anticorps reconnaissant spécifiquement le VEGF et la relaxine, et
- un kit comprenant au moins un anticorps reconnaissant spécifiquement la relaxine et le PIGF.

La présente invention vise également l'utilisation d'un tel kit pour diagnostiquer une cardiomyopathie peripartum (CMPP) chez une femme. Avantageusement, ce kit pourra servir à la mise en œuvre de la méthode de diagnostic de l'invention décrite ci-dessus (comprenant la mesure du taux de relaxine).

La présente invention vise par ailleurs l'utilisation d'un kit comprenant au moins un anticorps reconnaissant spécifiquementla relaxine et le PlGF dans un échantillon biologique pour diagnostiquer une cardiomyopathie peripartum (CMPP) chez une femme. Avantageusement, ce kit pourra servir à la mise en œuvre de la méthode de diagnostic de l'invention décrite ci-dessus (comprenant la mesure du ratio [relaxine/PlGF]).

La présente invention vise enfin l'utilisation d'un kit comprenant au moins un anticorps reconnaissant spécifiquement la relaxine et le VEGF dans un échantillon biologique pour diagnostiquer une cardiomyopathie peripartum (CMPP) chez une femme.

Avantageusement, ce kit pourra servir à la mise en œuvre de la méthode de diagnostic de l'invention décrite ci-dessus (comprenant la mesure du ratio [relaxine/VEGF]).

La présente invention vise enfin l'utilisation d'un kit comprenant les moyens de détecter et/ou de quantifier les taux de sFlt-1 et de PlGF dans un échantillon biologique pour diagnostiquer une cardiomyopathie peripartum (CMPP) chez une femme. Avantageusement, ce kit pourra servir à la mise en œuvre de la méthode de diagnostic de l'invention décrite ci-dessus (comprenant la mesure du ratio [sFlt-1/PlGF]).

Ces moyens de détection sont des anticorps reconnaissants spécifiquement le PlGF humain, le VEGF humain, le sFlt-1humain ou la relaxine humaine. Ces anticorps sont largement connus et disponibles commercialement. Ces moyens peuvent également être des amorces de PCR ou des sondes détectant les ARNm de ces protéines.

Ces kits peuvent également contenir des instructions pour mettre en œuvre les méthodes de l'invention, et/ou des échantillons de référence permettant d'étalonner les valeurs de référence utilisées aux étapes de comparaison.

### LEGENDES DES FIGURES

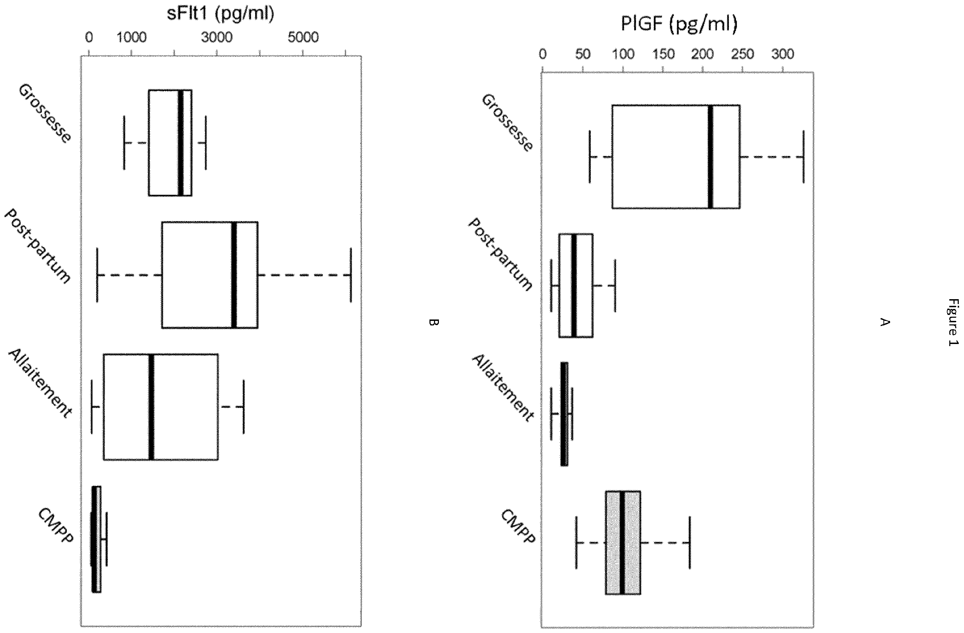

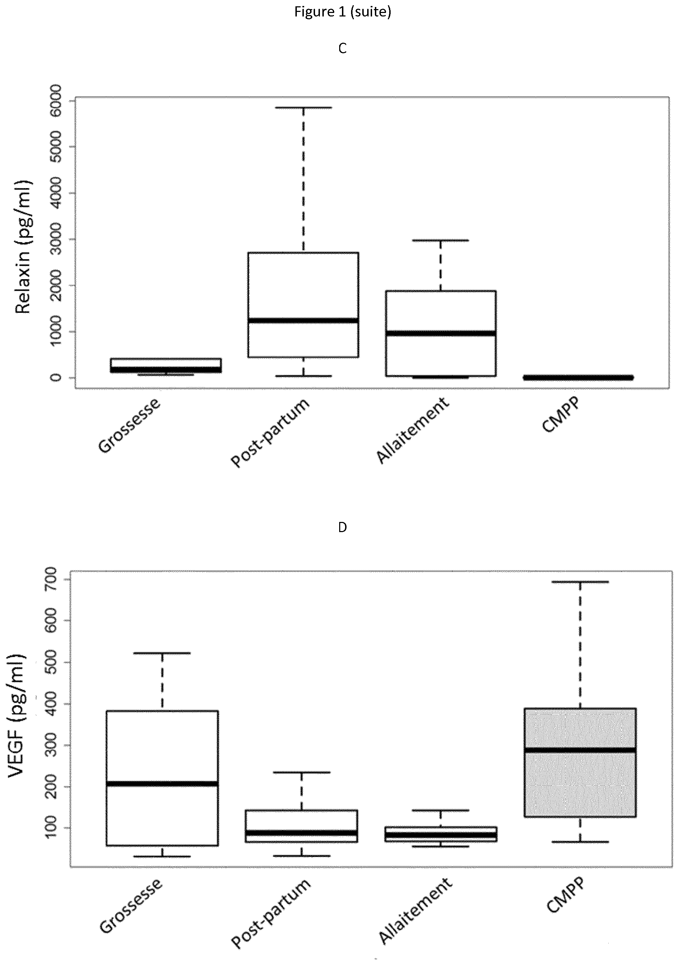

La **Figure 1** décrit les concentrations plasmatiques des biomarqueurs de l'invention pour des patientes souffrant de CMPP et des patientes non atteintes de CMPP, en particulier pour des femmes enceintes, au moment de l'accouchement, ou après l'accouchement pendant la période d'allaitement (allaitante). Les biomarqueurs dosés sont le PlGF (A), le sFlt-1 (B), la Relaxine (C) et le VEGF (D).

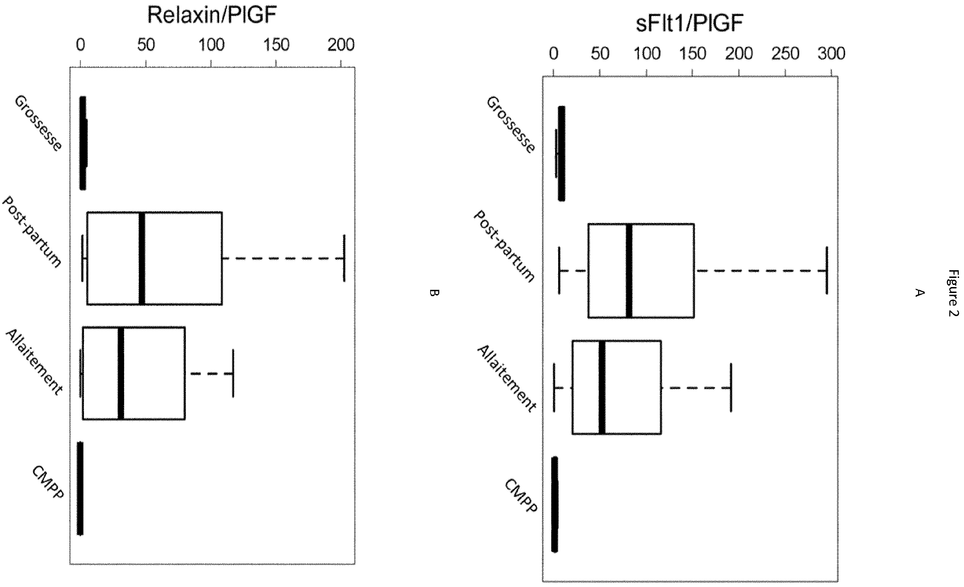

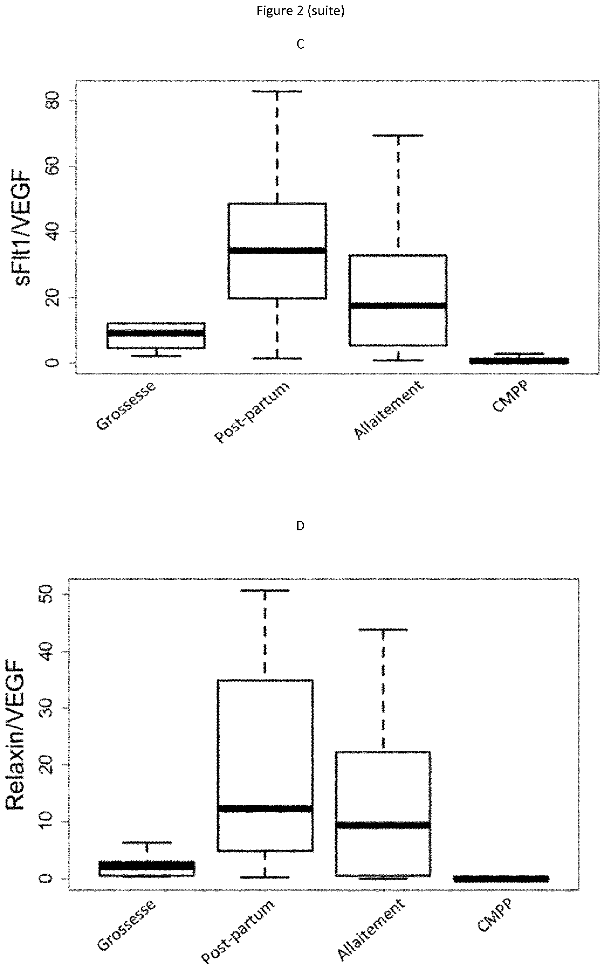

La **Figure 2** décrit les rapports de concentrations plasmatiques des biomarqueurs de l'invention pour des patientes souffrant de CMPP et des patientes non atteintes de CMPP, en particulier pour des femmes enceintes, au moment de l'accouchement, ou après l'accouchement pendant la période d'allaitement (allaitante). Les ratios représentés sont : le ratio sFlt-1/PlGF (A), le ratio Relaxine /PlGF (B), le ratio sFlt-1 / VEGF (C), et le ratio Relaxine / VEGF (D).

### EXEMPLES

### 1. Matériel et Méthodes.

La présente étude est une étude observationnelle prospective multicentrique regroupant des patientes d'Afrique du Sud (Chris Hani Baragwanath Hospital (CHB), Soweto) et de France (Hôpital Bichat et Hôpital Lariboisière, Paris). Cette étude a inclus de 2009 à ce jour, un total de 133 patientes réparties comme suit : 58 patientes ayant une cardiomyopathie du péri-partum dont 25 suivies à 3-9 mois après le diagnostic. Ces patientes ont été comparées à 75 femmes dans la phase du péri-partum appartenant à plusieurs sous-groupes : des femmes en fin de grossesse (n=10) ; des accouchées saines (40 patientes) ; des allaitantes saines (25 patientes)

Les critères d'inclusion des patientes ayant une CMPP étaient les suivants : âge ≥ 18 ans, symptômes d'insuffisance cardiaque aiguë dans le dernier mois de grossesse jusqu'au 5^{ème} mois du post-partum, absence d'autre cause identifiable d'insuffisance cardiaque, fraction d'éjection du ventricule gauche (FEVG) ≤ 45% mesurée par échocardiographie trans-thoracique.

Les critères d'inclusion des accouchées étaient les suivants: âge ≥ 18 ans, J0 d'accouchement.

Les critères d'inclusion des allaitantes étaient les suivants : âge ≥ 18 ans, allaitement en cours, dosage effectué un mois après l'accouchement.

Les critères d'exclusion des patientes ayant une cardiomyopathie du péri-partum étaient les suivants : antécédents cardiovasculaires, pré-eclampsie ou éclampsie en cours de grossesse, PAS > 160 mmHg ou PAD > 100 mmHg, autres conditions que la cardiomyopathie qui pourraient augmenter les marqueurs de l'inflammation, anémie sévère avec hémoglobine en début de grossesse < 9g/dl, tumeurs malignes, cardiopathie ischémique.

Les critères d'exclusion des accouchées et des allaitantes étaient les suivants : antécédents de cardiopathie connue, signes cliniques d'insuffisance cardiaque, accouchement compliqué d'un état de choc, pré-eclampsie ou éclampsie.

Les patientes ont été prises en charge en réanimation ou en unité de cardiologie (unité de soins intensifs de cardiologie ou salle en fonction de leur état clinique) par un réanimateur ou un cardiologue référent. Elles ont bénéficié à l'admission d'une évaluation clinique incluant un interrogatoire, l'évaluation du score NYHA, la mesure d'une pression artérielle non invasive après 30 minutes de repos et en position allongée et léger décubitus latéral gauche pour les femmes enceintes et de la fréquence cardiaque, d'une échocardiographie trans-thoracique en mode bidimensionnel avec doppler pour l'évaluation des données morphologiques et des fonctions systoliques et diastolique ventriculaires et d'un bilan biologique incluant une numération sanguine, une évaluation des fonctions rénale et hépatique, un dosage du cholestérol et des prélèvements spécifiques stockés .

Des prélèvements spécifiques à but de recherche ont été faits après obtention du consentement libre et éclairé des patientes et ce par prélèvements de 3 tubes EDTA (ethylènediaminetetra-acetic acid), centrifugés à 2500 tours/minutes pendant 7 minutes puis aliquotés dans des aliquots de 500 µl stockés à -80°C.

Des dosages ont été réalisés sous la direction de Dr JL. Samuel et Pr A. Mebazaa à l'unité Inserm 942 (Directeur Pr A. Cohen Solal) et en service de Biochimie de l'hôpital Lariboisière (Pr Launay). La relaxine a été dosée manuellement par méthode ELISA avec un anticorps spécifique (Xceltis, Allemagne) ; il a fallu au préalable vérifier la fiabilité des anticorps primaires et secondaires par l'utilisation des gammes d'étalonnage. VEGF et le sFLT1 ont été dosés sur automates (Abbott C3000).

Les caractéristiques de la population ont été exprimées en médiane ± interquartiles. Les comparaisons entre deux groupes ont été réalisées par un test non paramétrique de Mann Whithney, les comparaisons des groupes ont été réalisées par un test de Kruskal-Wallis et intergroupe par des post test Dunn's avec une significativité si p<0.05. La discrimination CMPP/non CMPP et le seuil pour les biomarqueurs et les ratios de biomarqueurs de l'invention ont été analysés par la méthode ROC.

### 2. Résultats

### 2.1. Caractéristiques des patientes étudiées

**Tableau 1. Caractéristiques des patientes présentant une cardiomyopathie du post-partum. FEVG : fraction d'éjection ventriculaire gauche ; PAS : pression artérielle systolique ; PAD : pression artérielle diastolique).**

| | **Enceintes (n=10)** | **A l'accouchement (n=40)** | **Allaitantes (n=25)** | **Toutes les CMPP (n=58)** |
|---|---|---|---|---|
| **Age (ans)** | 28 (25 to 38) | 33 (30 to 37) | 28 (23 to 30) | 27 (23 - 34) |
| **Parité** | | | | 2 (1 - 3) |
| **FEVG (%)** | | | | 30 (22- 34) |
| **NYHA class n(%)** | | | | |
| **I** | | | | 0 (0) |
| **II** | | | | 12 (50) |
| **III** | | | | 7 (29) |
| **IV** | | | | 5 (21) |
| **PAS (mmHg)** | | | | 112 (109 - 126) |
| **PAD (mmHg)** | | | | 72 (68 - 80) |

### 2.2. Variation des taux de marqueurs pro- et anti-angiogéniques :

2.2.1. Les résultats des dosages pour les différents marqueurs de l'invention sont décrits dans les tableaux 2, 3 et 4 ci-dessous:

**Tableau 2. Niveaux des biomarqueurs plasmatiques dans les différents sous-groupes**

| | **Enceintes (n=10)** | **A l'accouchement (n=40)** | **Allaitantes (n=25)** | **CMPP (n=58)** |
|---|---|---|---|---|
| **PlGF (pg/mL)** | 210 (117 - 242) | 39 (21 - 59) | 26 (23 - 31) | 100 (80 - 122) |
| **sFlt1 (pg/mL)** | 2156 (1416 - 2410) | 3404 (1722 - 3941) | 1468 (351 - 3020) | 132 (99 - 278) |
| **Relaxine pg/ml** | 171 (121 - 347) | 1242 (490 - 2556) | 957 (33 - 1865) | 0 (0 - 4) |
| **VEGF pg/ml** | 207 (82 - 345) | 88 (67 - 143) | 83 (68 - 102) | 289 (131 - 389) |
| **sFlt1/P1GF** | 8.8 (6.9 to 11.2) | 81.6 (37.4 to 151) | 52.8 (20.6 to 116.2) | 1.3 (0.9 to 2.8) |
| **Relaxine/P1GF** | 1.3 (0.6 to 3.1) | 46.9 (5.2 to 107.8) | 31.1 (1.9 to 80.2) | 0 (0 to 0) |
| **sFlt1/VEGF** | 9 (4.4 to 12.1) | 34.2 (19.8 to 48.5) | 17.5 (5.3 to 32.7) | 0.6 (0.3 to 1.3) |
| **Relaxine/VEGF** | 2 (0.9 to 2.7) | 12.3 (4.8 to 34.4) | 9.4 (0.5 to 22.3) | 0 (0 to 0) |

**Tableau 3. Performances diagnostiques des différents biomarqueurs de l'invention**

| | **Péripartum Non-CMPP (n=75)** | **CMPP (n=58)** | **p value** |
|---|---|---|---|
| **PlGF (pg/mL)** | 32 (23 to 76) | 100 (80 - 122) | <0.0001 |
| **sFlt1 (pg/mL)** | 2439 (1352 to 3546) | 132 (99 to 278) | <0.0001 |
| **Relaxine pg/ml** | 957 (33 - 1865) | 0 (0 to 3.6) | <0.0001 |
| **VEGF pg/ml** | 88 (66.5 to 142.7) | 289 (131 to 389) | <0.0001 |
| **sFlt1/P1GF** | 54.4 (20.7 to 124) | 1.3 (0.9 to 2.8) | <0.0001 |
| **Relaxine/P1GF** | 30.6 (3.6 to 84.0) | 0 (0 to 0) | <0.0001 |
| **sFlt1/VEGF** | 23.5 (9.3 to 44.2) | 0.6 (0.3 to 1.3) | <0.0001 |
| **Relaxine/VEGF** | 8.82 (2.65 to 21.08) | 0 (0 to 0.01) | <0.0001 |

Le tableau 3 décrit les concentrations plasmatiques des différents biomarqueurs de l'invention. Ces résultats démontrent qu'il est possible, grâce à ces biomarqueurs, de différencier le groupe des femmes ayant développé une CMPP de celui des femmes saines, i.e., en péripartum n'ayant pas développé une CMPP (ce groupe regroupe les 3 sous-groupes suivants : enceintes (n=10), femmes venant d'accoucher (n=40), femmes allaitantes (n=25) cf tableau 1 et 2). Pour le groupe CMPP, les prélèvements ont été faits au moment du diagnostic.

**Tableau 4. Performances diagnostiques des différents biomarqueurs de l'invention**

| | **AUC [95% IC]** | **Seuil** | **Spé** | **Se** | **VPN** | **VPP** |
|---|---|---|---|---|---|---|
| **PlGF (pg/mL)** | 0.837 [0.759 - 0.838] | 61 | 0.72 | 0.95 | 0.95 | 0.72 |
| **sFlt1 (pg/mL)** | 0.903 [0.841 - 0.905] | 481 | 0.86 | 0.95 | 0.95 | 0.84 |
| **Relaxine (pg/ml)** | 0.950 [0.903 - 0.951] | 29 | 0.89 | 0.95 | 0.96 | 0.87 |
| **VEGF (pg/ml)** | 0.855 [0.786 - 0.858] | 119 | 0.71 | 0.84 | 0.85 | 0.69 |
| **sFlt1/P1GF** | 0.956 [0.919 - 0.958] | 5.1 | 0.90 | 0.93 | 0.94 | 0.88 |
| **Relaxine/P1GF** | 0.965 [0.916 - 0.967] | 0.4 | 0.93 | 0.95 | 0.98 | 0.82 |
| **sFlt1/VEGF** | 0.962 [0.932 - 0.963] | 6.6 | 0.89 | 0.95 | 0.96 | 0.87 |
| **Relaxine/VEGF** | 0.962 [0.914 - 0.964] | 0.2 | 0.93 | 0.95 | 0.98 | 0.82 |

Le tableau 4 décrit les AUC (pour « Area under the curve »), l'intervalle de confiance (IC), la sensibilité (Se), la spécificité (Spé) et les valeurs seuils, prédictive négative (VPN) et prédictive positive (VPP) des différents biomarqueurs de l'invention (lors des expériences ayant mené aux résultats du tableau 3). Ces résultats ont permis de définir le seuil de discrimination entre CMPP et non-CMPP par la technique de la distance la plus courte.

Ces résultats confirment le fait qu'il est possible de diagnostiquer des patientes souffrant de CMPP, grâce aux biomarqueurs PlGF, VEGF, Relaxine, et sFlt-1 seuls, ou, mieux encore, grâce aux ratios sFlt-1/PlGF, Relaxine/PlGF, sFlt-1/VEGF ou Relaxine/VEGF.

### 2.2.2. sFlt-1 et les rapports s-Flt-1/PlGF ou sFlt-1/VEGF

La concentration plasmatique de sFlt1 (Tableaux 2 et 3) chez les CMPP est de 132 pg/ml (99 pg/ml -278 pg/ml). Elle est inférieure à celle des femmes enceintes, des accouchées ou des allaitantes saines (tableaux 2 et 3).

Le rapport sFlt-1/PlGF est de 1,32 (0.93 - 2,78) chez les CMPP, plus bas que les rapports sFlt-1/PlGF chez des femmes enceintes, des accouchées ou des allaitantes saines (tableaux 2 et 3).

Ce résultat démontre la prédominance de l'angiogenèse chez les CMPP. Cette prédominance est particulièrement confirmée par le rapport sFlt-1/PlGF très bas dans les CMPP. En effet l'AUC (facteur de discrimination) des CMPP par rapport aux femmes saines dans la phase du péripartum est de 0,956 [0,918-0,957] (Tableau 4). De plus le rapport sFlt-1/PlGF a une spécificité et une sensibilité pour le diagnostic de CMPP, tous les 2 supérieurs à 0,90 (Tableau 4).

La prédominance de l'angiogenèse est également confirmée par le rapport sFlt-1/VEGF, également très bas chez les CMPP et l'AUC, la spécificité et la sensibilité très élevées du rapport sFlt-1/VEGF chez les CMPP (Tableaux 3 et 4).

### 2.2.3. Relaxine

La concentration plasmatique de relaxine est très bas voire indétectable chez les CMPP : 0 (0 - 3,6) pg/ml) exprimée en médiane (25^{ème} -75^{ème}). Elle est nettement inférieure à celle des femmes enceintes, des accouchées et des allaitantes saines, respectivement de 171 pg/ml (121 pg/ml - 347 pg/ml), 1242 pg/ml (490 pg/ml - 2556 pg/ml, p<0,0001) et 957 pg/ml (33 pg/ml - 1865 pg/ml (p<0,0001).

Chez les CMPP suivies plusieurs mois après le diagnostic (n=20), la concentration plasmatique de relaxine est demeurée basse au cours du suivi de plusieurs mois à 2,56 pg/ml (0 pg/ml - 8,62 pg/ml, p = NS) ; le rapport sFlt-1/PlGF est également demeuré stable.

### 3. Discussion

Les dosages plasmatiques des biomarqueurs de l'angiogenèse réalisés à l'admission en cardiologie ou soins intensifs chez les femmes ayant une CMPP sont en faveur d'une altération profonde des éléments majeurs de la balance angiogénique. La présente étude montre que la relaxine, le sFlt1 et les rapports sFlt-1/PlGF ou sFlt-1/VEGF, sont fortement diminués dans le plasma des CMPP.

Il ressort de cette étude que la baisse de la concentration plasmatique de la relaxine est associée à une élévation du PlGF ou du VEGF chez les CMPP. Ce résultat peut paraitre paradoxal car la relaxine a été décrite comme stimulant la sécrétion de VEGF (Unemori EN et al. Hum Reprod. 1999 Mar;14(3):800-6). Il est possible que ce résultat puisse s'expliquer par un rétrocontrôle négatif du VEGF sur la relaxine.

L'élévation de VEGF est également associée à une baisse importante de sFLt1. Ceci est compréhensible puisque le sFlt1 est le récepteur circulant de plusieurs facteurs angiogéniques dont le VEGF (Bdolah Y et al. Semin Nepbrol 24, 548-556, 2004). Dans la cohorte étudiée, la baisse du sFlt1 pourrait donc être l'élément déclenchant de la perturbation de la balance angiogénique chez les CMPP.

Les données de la littérature antérieurement publiées rapportent une augmentation du sFlt-1 plasmatique chez les femmes enceintes. Ce taux circulant de sFlt-1 est encore plus élevé au cours des pré-eclampsies dépassant les 10000 pg/ml aboutissant à un rapport sFlt-1/PlGF très élevé. I.S. Patten et al a rapporté dans leur série de patientes CMPP une augmentation du taux de sFlt-1 (Patten IS et al., Nature 2012). Néanmoins, ces patientes étaient pré-éclamptiques dans 40 % des cas.

Dans la présente étude, les parturientes préalablement pré-éclamptiques n'ont pas été incluses.

## Revendications

1. Méthode pour diagnostiquer une cardiomyopathie du peripartum (CMPP) chez une femme non pré-éclamptique, ladite méthode comprenant les étapes de :
a) mesurer *in vitro,* dans un échantillon de sang de ladite femme, le taux de VEGF, le PlGF, le sFlt-1 et/ou la relaxine,
b) comparer ce(s) taux à une (des) valeur(s) de référence,
dans laquelle si le taux de PlGF mesuré à l'étape a) est supérieur à la valeur de référence pour ce marqueur, et/ou si le taux de VEGF est supérieur à la valeur de référence pour ce marqueur, et/ou si le taux de sFlt-1 mesuré à l'étape a) est inférieur à la valeur de référence pour ce marqueur, et/ou si le taux de relaxine mesuré à l'étape a) pour ce marqueur est supérieur à la valeur de référence pour ce marqueur, alors cette femme souffre d'une CMPP.

2. Méthode selon la revendication 1, comprenant les étapes :
a) mesurer *in vitro* le taux de VEGF et/ou de PlGF dans un échantillon de sang de ladite femme,
b) comparer ce(s) taux à une (des) valeur(s) de référence,
dans laquelle un taux de VEGF et/ou de PlGF mesuré à l'étape a) supérieur à ladite valeur de référence indique que ladite femme souffre d'une CMPP.

3. Méthode selon la revendication 1, comprenant les étapes :
a) mesurer *in vitro* le taux de sFlt-1 dans un échantillon de sang de ladite femme,
b) comparer ce(s) taux à une (des) valeur(s) de référence,
dans laquelle un taux de sFlt-1 mesuré à l'étape a) inférieur à ladite valeur de référence indique que cette femme souffre d'une CMPP.

4. Méthode selon la revendication 1, comprenant les étapes :
a) mesurer *in vitro* les taux de VEGF et de relaxine dans un échantillon de sang de ladite femme,
b) calculer le ratio [relaxine/VEGF] et le comparer à une valeur de référence,
dans laquelle un ratio calculé à l'étape b) inférieur à la valeur de référence pour ce ratio ou nul indique que ladite femme souffre d'une CMPP.

5. Méthode selon l revendication 1, comprenant les étapes :
a) mesurer *in vitro* les taux de PlGF et de relaxine dans un échantillon de sang de ladite femme,
b) calculer le ratio [relaxine/PlGF] et le comparer à une valeur de référence
dans laquelle un ratio calculé à l'étape b) inférieur à la valeur de référence pour ce ratio ou nul indique que ladite femme souffre d'une CMPP.

6. Méthode selon les revendications 1, comprenant les étapes :
a) mesurer *in vitro* le taux de PlGF et de sFlt-1 dans un échantillon de sang de ladite femme,
b) calculer le ratio [sFlt-l/PlGF] et le comparer à une valeur de référence,
dans laquelle un ratio [sFlt-l/PlGF] calculé à l'étape b) inférieur à ladite valeur de référence pour ce ratio indique que ladite femme souffre d'une CMPP.

7. Méthode selon la revendication 1, comprenant les étapes :
a) mesurer *in vitro* le taux de VEGF et de sFlt-1 dans un échantillon de sang de ladite femme,
b) calculer le ratio [sFlt-l/VEGF] et le comparer à une valeur de référence,
dans laquelle un ratio [sFlt-l/VEGF] calculé à l'étape b) inférieur à ladite valeur de référence pour ce ratio indique que ladite femme souffre d'une CMPP.

8. Méthode selon l'une quelconque des revendications 1 à 7, **caractérisée en ce que** ladite femme est enceinte, de préférence dans le troisième trimestre de sa grossesse.

9. Méthode selon l'une quelconque des revendications 1 à 7, **caractérisée en ce que** ladite femme a accouché dans les mois précédent le prélèvement de l'échantillon.

10. Méthode selon l'une quelconque des revendications 1 à 9, **caractérisée en ce que** ledit échantillon de sang est du plasma sanguin ou du sérum sanguin.

11. Kit comprenant au moins un anticorps reconnaissant spécifiquement le VEGF et la relaxine.

12. Kit comprenant au moins un anticorps reconnaissant spécifiquement la relaxine et le PlGF.

13. Utilisation des kits selon les revendications 11 ou 12, pour diagnostiquer une cardiomyopathie du peripartum (CMPP) chez une femme selon les méthodes décrites aux revendications 1 à 7.

## Patentansprüche

1. Verfahren zur Diagnose einer peripartalen Kardiomyopathie (PPCM) bei einer Frau ohne Präeklampsie, wobei das Verfahren die Schritte umfasst:
a) Messen des Gehalts an VEGF, PlGF, sFlt-1 und/oder Relaxin in einer Blutprobe von der Frau *in vitro,*
b) Vergleichen dieses Gehalts bzw. dieser Gehalte mit einem bzw. mehreren Referenzwerten,
wobei, wenn der im Schritt a) gemessene Gehalt an PlGF höher als der Referenzwert für diesen Marker ist und/oder wenn der Gehalt an VEGF höher als der Referenzwert für diesen Marker ist und/oder wenn der im Schritt a) gemessene Gehalt an sFlt-1 kleiner als der Referenzwert für diesen Marker ist und/oder wenn der im Schritt a) gemessene Gehalt an Relaxin höher als der Referenzwert für diesen Marker ist, diese Frau an einer PPCM leidet.

2. Verfahren nach Anspruch 1, umfassend die Schritte:
a) Messen des Gehalts an VEGF und/oder PlGF in einer Blutprobe von der Frau *in vitro,*
b) Vergleichen dieses Gehalts bzw. dieser Gehalte mit einem bzw. mehreren Referenzwerten,
wobei ein im Schritt a) gemessener Gehalt an VEGF und/oder PlGF, der höher als der Referenzwert ist, angibt, dass die Frau an einer PPCM leidet.

3. Verfahren nach Anspruch 1, umfassend die Schritte:
a) Messen des Gehalts an sFlt-1 in einer Blutprobe von der Frau *in vitro,*
b) Vergleichen dieses Gehalts bzw. dieser Gehalte mit einem bzw. mehreren Referenzwerten,
wobei ein im Schritt a) gemessener Gehalt an sFlt-1, der kleiner als der Referenzwert ist, angibt, dass diese Frau an einer PPCM leidet.

4. Verfahren nach Anspruch 1, umfassend die Schritte:
a) Messen der Gehalte an VEGF und Relaxin in einer Blutprobe von der Frau *in vitro,*
b) Berechnen des Verhältnisses [Relaxin/VEGF) und Vergleichen dieses mit einem Referenzwert,
wobei ein im Schritt b) berechnetes Verhältnis, das kleiner als der Referenzwert für dieses Verhältnis oder null ist, angibt, dass die Frau an einer PPCM leidet.

5. Verfahren nach Anspruch 1, umfassend die Schritte:
a) Messen der Gehalte an PlGF und Relaxin in einer Blutprobe von der Frau *in vitro,*
b) Berechnen des Verhältnisses [Relaxin/PlGF) und Vergleichen dieses mit einem Referenzwert,
wobei ein im Schritt b) berechnetes Verhältnis, das kleiner als der Referenzwert für dieses Verhältnis oder null ist, angibt, dass die Frau an einer PPCM leidet.

6. Verfahren nach Anspruch 1, umfassend die Schritte:
a) Messen des Gehalts an PlGF und sFlt-1 in einer Blutprobe von der Frau *in vitro,*
b) Berechnen des Verhältnisses [sFlt-1/PlGF] und Vergleichen dieses mit einem Referenzwert,
wobei ein im Schritt b) berechnetes Verhältnis [sFlt-1/PlGF], das kleiner als der Referenzwert für dieses Verhältnis ist, angibt, dass die Frau an einer PPCM leidet.

7. Verfahren nach Anspruch 1, umfassend die Schritte:
a) Messen des Gehalts an VEGF und sFlt-1 in einer Blutprobe von der Frau *in vitro,*
b) Berechnen des Verhältnisses [sFlt-1/VEGF] und Vergleichen dieses mit einem Referenzwert,
wobei ein im Schritt b) berechnetes Verhältnis [sFlt-1/VEGF), das kleiner als der Referenzwert für dieses Verhältnis ist, angibt, dass die Frau an einer PPCM leidet.

8. Verfahren nach einem der Ansprüche 1 bis 7, **dadurch gekennzeichnet, dass** die Frau schwanger ist, vorzugsweise im dritten Trimester ihrer Schwangerschaft.

9. Verfahren nach einem der Ansprüche 1 bis 7, **dadurch gekennzeichnet, dass** die Frau in dem Monat, der der Entnahme der Probe vorausgeht, entbunden hat.

10. Verfahren nach einem der Ansprüche 1 bis 9, **dadurch gekennzeichnet, dass** die Blutprobe von Blutplasma oder Blutserum ist.

11. Kit, umfassend mindestens einen Antikörper, der VEGF und Relaxin spezifisch erkennt.

12. Kit, umfassend mindestens einen Antikörper, der Relaxin und PlGF spezifisch erkennt.

13. Verwendung von Kits nach den Ansprüchen 11 oder 12 zur Diagnose einer peripartalen Kardiomyopathie (PPCM) bei einer Frau gemäß den in den Ansprüchen 1 bis 7 beschriebenen Verfahren.

## Claims

1. A method for diagnosing peripartum cardiomyopathy (PPCM) in a non-pre-eclamptic woman, said method comprising the steps of:
a) measuring *in vitro* the level of VEGF, PlGF, sFlt-1 and/or relaxin in a blood sample from said woman,
b) comparing the level(s) to one or more reference value(s),
wherein if the level of PlGF measured in step a) is higher than the reference value for that marker, and/or if the level of VEGF is higher than the reference value for that marker, and/or if the level of sFlt-1 measured in step a) is lower than the reference value for that marker, and/or if the level of relaxin measured in step a) for that marker is higher than the reference value for that marker, then that woman is suffering from PPCM.

2. The method as claimed in claim 1, comprising the steps of:
a) measuring *in vitro* the level of VEGF and/or PlGF in a blood sample from said woman,
b) comparing the levels(s) to one or more reference value(s),
wherein a level of VEGF and/or of PlGF measured in step a) higher than said reference value indicates that said woman is suffering from PPCM.

3. The method as claimed in claim 1, comprising the steps of:
a) measuring *in vitro* the level of sFlt-1 in a blood sample from said woman,
b) comparing the rate(s) to one or more reference value(s),
wherein a level of sFlt-1 measured in step a) lower than said reference value indicates that the woman is suffering from PPCM.

4. The method as claimed in claim 1, comprising the steps of:
a) measuring *in vitro* the levels of VEGF and relaxin in a blood sample from said woman,
b) calculating the [relaxin/VEGF] ratio and comparing it with a reference value,
wherein a ratio calculated in step b) lower than the reference value for that ratio or zero indicates that said woman is suffering from PPCM.

5. The method as claimed in claim 1, comprising the steps of:
a) measuring *in vitro* the levels of PlGF and relaxin in a blood sample from said woman,
b) calculating the [relaxin/PlGF] ratio and comparing it with a reference value
wherein a ratio calculated in step b) lower than the reference value for that ratio or zero indicates that said woman is suffering from PPCM.

6. The method as claimed in claim 1, comprising the steps of:
a) measuring *in vitro* the level of PlGF and of sFlt-1 in a blood sample from said woman,
b) calculating the [sFlt-1/PIGF] ratio and comparing it with a reference value,
wherein a [sFlt-1/PlGF] ratio calculated in step b) lower than said reference value for that ratio indicates that said woman is suffering from PPCM.

7. The method as claimed in claim 1, comprising the steps of:
a) measuring *in vitro* the level of VEGF and of sFlt-1 in a blood sample from said woman,
b) calculating the [sFlt-1/VEGF] ratio and comparing it with a reference value,
wherein a [sFlt-1/VEGF] ratio calculated in step b) lower than said reference value for that ratio indicates that said woman is suffering from PPCM.

8. The method as claimed in any one of claims 1 to 7, **characterized in that** said woman is pregnant, preferably in the third trimester of her pregnancy.

9. The method as claimed in any one of claims 1 to 7, **characterized in that** said woman has given birth within the months preceding the sample collection.

10. The method as claimed in any one of claims 1 to 9, **characterized in that** said blood sample is blood plasma or blood serum.

11. A kit comprising at least one antibody specifically recognizing VEGF and relaxin.

12. A kit comprising at least one antibody specifically recognizing relaxin and PIGF.

13. Use of the kits as claimed in claim 11 or 12, for diagnosing peripartum cardiomyopathy (PPCM) in a woman according to the methods described in claim 1 to 7.
